# EUROPEAN PATENT APPLICATION

(11) **EP 4 190 334 A1**
(43) Date of publication of application: **07.06.2023**
(21) Application number: 22207362.9
(22) Date of filing: 14.06.2019
(51) Int. Cl.: A61K 31/7004, A61K 31/7008, A61K 31/7012, A61K 31/7072, A61K 45/06, A61P 21/00

(54) **METHODS AND MATERIALS FOR TREATING GLYCOSYLATION DISORDERS**

(30) Priority: 14.06.2018 US 201862685077 P
(62) Divisional of application: 19819212.2
(71) Applicant: Mayo Foundation for Medical Education and Research, Rochester, Minnesota 55905 (US); VIB VZW, 9052 Gent (BE); Katholieke Universiteit Leuven, K.U.Leuven R&D, 3000 Leuven (BE)
(72) Inventor: MORAVA-KOZICZ, Eva, Rochester, MN 55902 (US); KOZICZ, Tamas, Rochester, MN 55902 (US); GHESQUIÈRE, Bart, 3061 Leefdaal (BE)
(74) Representative: Fish & Richardson P.C.

(57) **Abstract**

This document provides methods and materials involved in treating congenital disorders of glycosylation (CDGs). For example, methods for using a composition including one or more uridine diphosphate (UDP) - sugars to treat a mammal (e.g., a human) having a CDG are provided.

## Description

### CROSS-REFERENCE TO RELATED APPLICATIONS

This application claims the benefit of U.S. Patent Application Serial No. 62/685,077, filed on June 14, 2018. The disclosure of the prior application is considered part of (and is incorporated by reference in) the disclosure of this application.

### BACKGROUND

### 1. Technical Field

This document relates to methods and materials involved in treating congenital disorders of glycosylation (CDGs). For example, this document provides methods and materials (e.g., compositions) for using one or more uridine diphosphate (UDP) - sugars to treat a mammal (e.g., a human) having a CDG.

### 2. Background Information

Congenital disorders of glycosylation (CDGs) are caused by defects in glycoprotein and glycolipid synthesis (Jaeken et al., 2011 J Inherit Metab Dis 34:853-858). There is no proven effective treatment available in most CDG types.

### SUMMARY

This document provides methods and materials for treating a mammal (e.g., a human) having a CDG. For example, a composition including one or more UDP-sugars can be used as described herein to treat a human having a CDG. In some cases, a composition including UDP-galactose and glucose or a derivative thereof (e.g., UDP-glucose) can be administered to a human having a CDG caused by a phosphoglucomutase 1 deficiency (e.g., a PGM1-CDG) to reduce the severity of the CDG and/or to reduce one or more symptoms of the CDG.

As demonstrated herein, treating PGM1-CDG patients with oral galactose restores depleted levels of UDP-galactose and UDP-glucose, thereby restarting stalled glycosylation in these patients. The ability to treat a PGM1-CDG patient by administering UDP-galactose in the presence of glucose or a derivative thereof (e.g., UDP-glucose) can allow clinicians and patients to safely and efficiently correct the PGM1-CDG phenotype.

In general, one aspect of this document features methods for treating a mammal having a CDG. The methods can include, or consist essentially of, administering a composition including UDP-galactose and UDP-glucose to a mammal having a CDG. The mammal can be a human. The CDG can be a phosphoglucomutase 1 CDG (PGM1-CDG). The ratio of UDP-galactose to UDP-glucose can be from about 1:1 to about 10:1 (e.g., 3:1). Administering the composition can result in the mammal having a blood concentration of UDP-galactose of from about 5 µM to about 50 µM. Administering the composition can result in the mammal having a blood concentration of UDP-glucose of from about 2 µM to about 20 µM. The treatment can be effective to reduce one or more symptoms of the CDG. The treatment can be effective to restore glycosylation in the mammal.

In another aspect, this document features methods for restoring glycosylation in a mammal having a CDG. The methods can include, or consist essentially of, administering a composition comprising UDP-galactose and UDP-glucose to a mammal having a CDG. The mammal can be a human. The CDG can be a PGM1-CDG. The ratio of UDP-galactose to UDP-glucose can be from about 1:1 to about 10:1 (e.g., 3:1). Administering the composition can result in the mammal having a blood concentration of UDP-galactose of from about 5 µM to about 50 µM. Administering the composition can result in the mammal having a blood concentration of UDP-glucose of from about 2 µM to about 20 µM. Restored glycosylation can be assessed using a ratio of a reduced transferrin glycosylation form over a normal transferrin glycosylation form, and a decreased ratio can indicate restored glycosylation. Restored glycosylation can be assessed using a level of one or more liver enzymes within the mammal, where a decreased level of one or more liver enzymes (e.g., aspartate transaminase (AST) and/or alanine transaminase (ALT)) can indicate restored glycosylation. Restored glycosylation can be assessed using a level of one or more coagulation factors within the mammal, where a decreased level of one or more coagulation factors (e.g., activated prothrombin time (aPTT)) can indicate restored glycosylation. Restored glycosylation can be assessed using a level of one or more coagulation factors within the mammal, where an increased level of one or more coagulation factors (e.g., anti-thrombin III (ATIII), Factor XI, and/or Factor XIII) can indicate restored glycosylation.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DESCRIPTION OF THE DRAWINGS

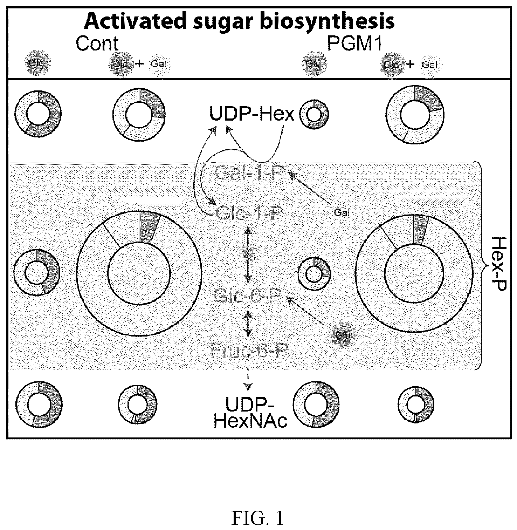

Figure 1 shows a schematic representation of an activated sugar biosynthesis.

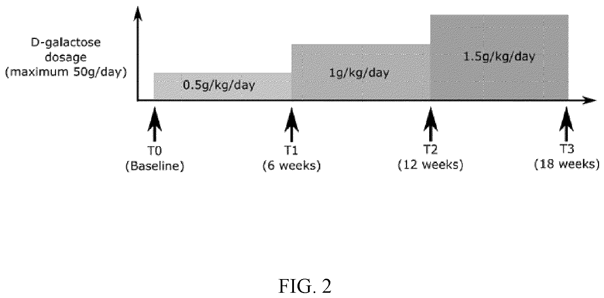

Figure 2 shows a schematic representation of an exemplary D-gal dosing schedule.

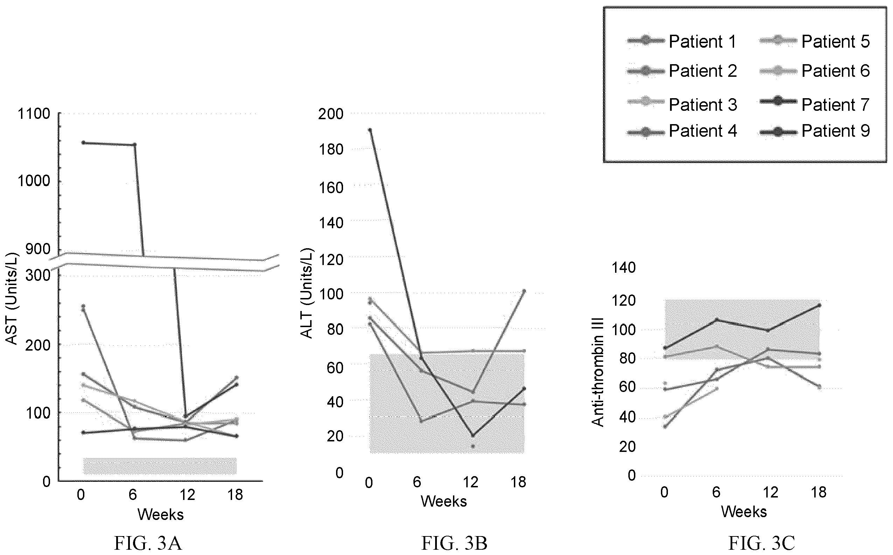

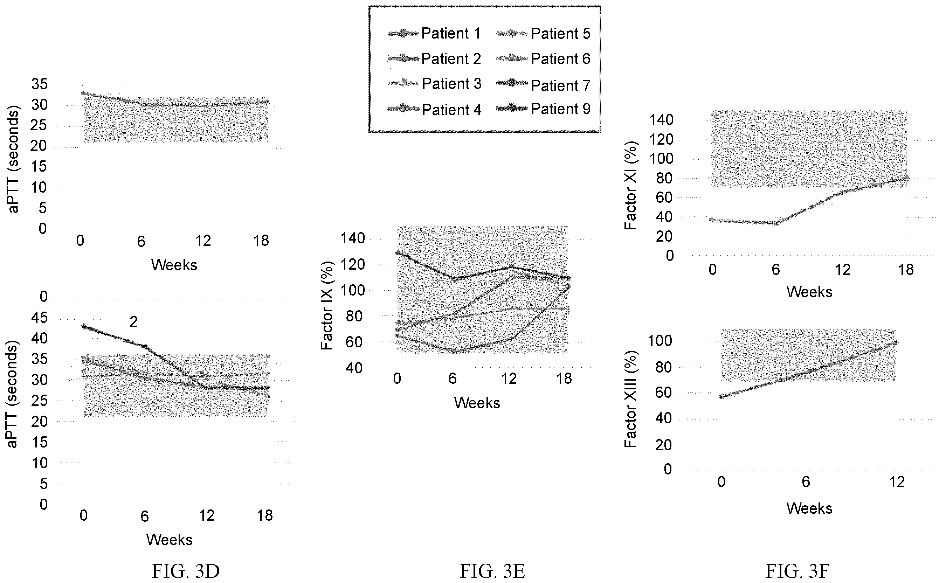

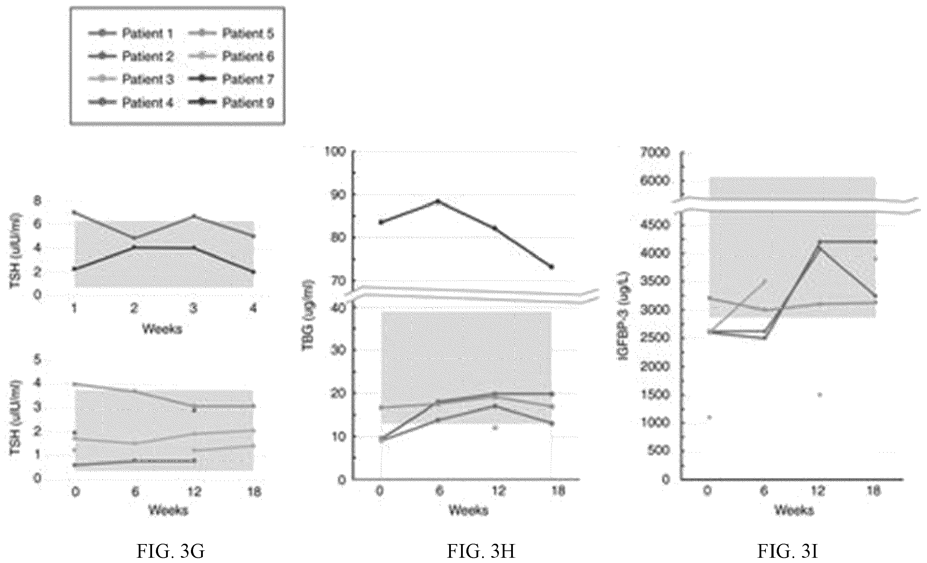

Figures 3A-3I show effects of D-gal supplementation. Figure 3A shows the effect of D-gal supplementation on aspartate transaminase (AST). Figure 3B shows the effect of D-gal supplementation on alanine transaminase (ALT). Figure 3C shows the effect of D-gal supplementation on anticoagulation (anti-thrombin III (ATIII)). Figures 3D-3F shows the effect of D-gal supplementation on coagulation (activated prothrombin time (aPTT) in Fig. 3D, Factor IX in Fig. 3E, and Factor XI and Factor XIII in Fig. 3F). Figure 3G shows the effect of D-gal supplementation on thyroid stimulating hormone (TSH). Figure 3H shows the effect of D-gal supplementation on thyroxine-binding globulin (TBG). Figure 3I shows the effect of D-gal supplementation on insulin-like growth factor-binding protein 3 (IGFBP-3). The shadowed areas represent the reference range.

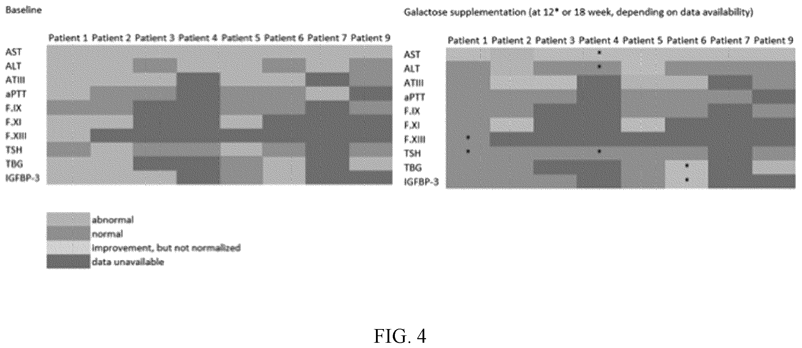

Figure 4 contains a scheme illustrating the effect of galactose supplementation on liver function enzymes, hemostatic factors, and endocrine proteins. The hemostatic and endocrine factors of interest are known glycoproteins. Orange, abnormal level; green, normal level; light green, improvement comparing to baseline, but did not reach normal level; grey, data not available. The measurement while on therapy was taken at either 12 weeks or 18 weeks, depending on data availability. Data taken at 12 weeks are denoted with an asterix*. With a few exceptions, in general glycoproteins that were abnormal at baseline improved or normalized at by 12 or 18 weeks.

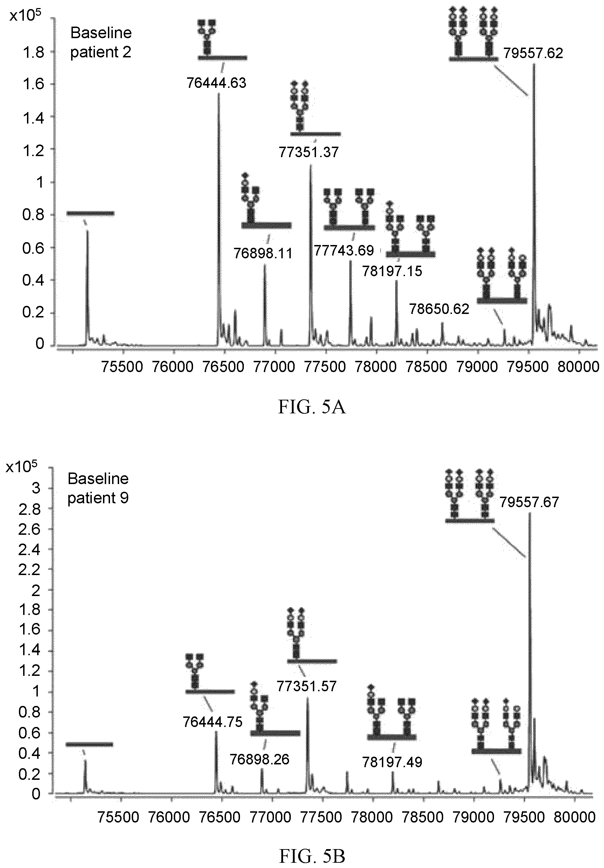

Figures 5A-5D show high-resolution mass spectrometry spectra of intact serum transferrin. Figures 5A and 5B show baseline profiles having characteristic PGM1 glycoforms with truncated glycans and lack of whole glycans. Figure 5A shows a baseline profile of patient 2. Figure 5B shows a baseline profile of patient 9. Patient 9 shows a milder profile than patient 2. Figures 5A and 5B show improvement through the reduction of abnormal glycosylation peaks upon 18 weeks of galactose treatment, as is highlighted by the green arrows. Figure 5A shows an improved profile of patient 2. Figure 5B shows an improved profile of patient 9.

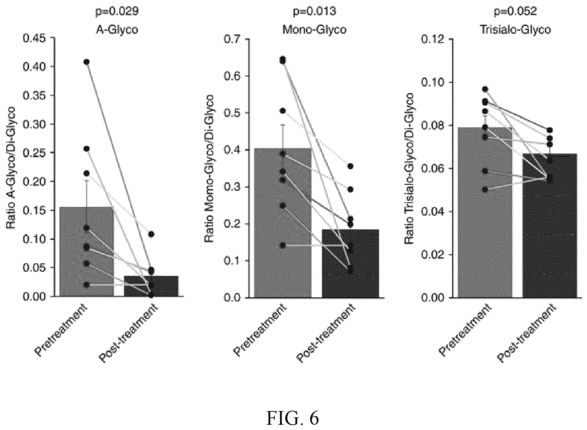

Figure 6 contains graphs showing high-resolution transferrin glycosylation analysis in PGM1-CDG patients before and after D-gal supplement. Ratios of nonglycosylated (A-Glyco) transferrin, monoglycosylated (Mono-Glyco) transferrin, and trisialo (Trisialo-Glyco) transferrin over normal (tetra-sialo or Di-glyco) transferrin were calculated and compared with reference ranges (repeated measures of analysis of variance). A-Glyco: ≤ 0.01040, Mono-Glyco: ≤ 0.02700, and Trisialo-Glyco: ≤ 0.031900.

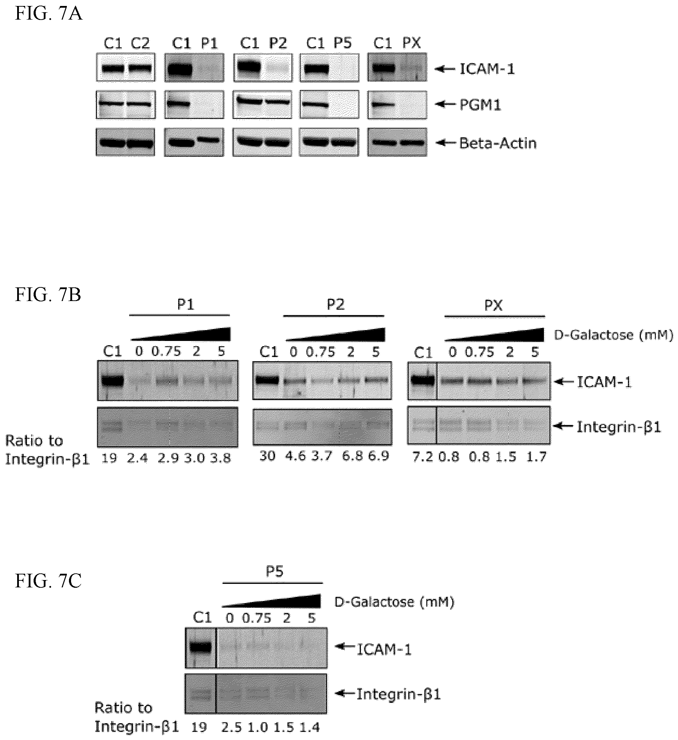

Figures 7A-7C contain analyses of PGM1 protein expression. Figure 7A shows protein expression of PGM1 and ICAM-1 in the skin fibroblasts of patients 1, 2, 5, and cell-line 2015X. Figure 7B shows improvement in ICAM-1 protein expression following galactose supplementation observed in the skin fibroblasts of patient 1, 2, and cell-line 2015X. Figure 7C shows a lack of improvement in ICAM-1 protein expression following galactose supplementation observed in the skin fibroblasts of patient 5.

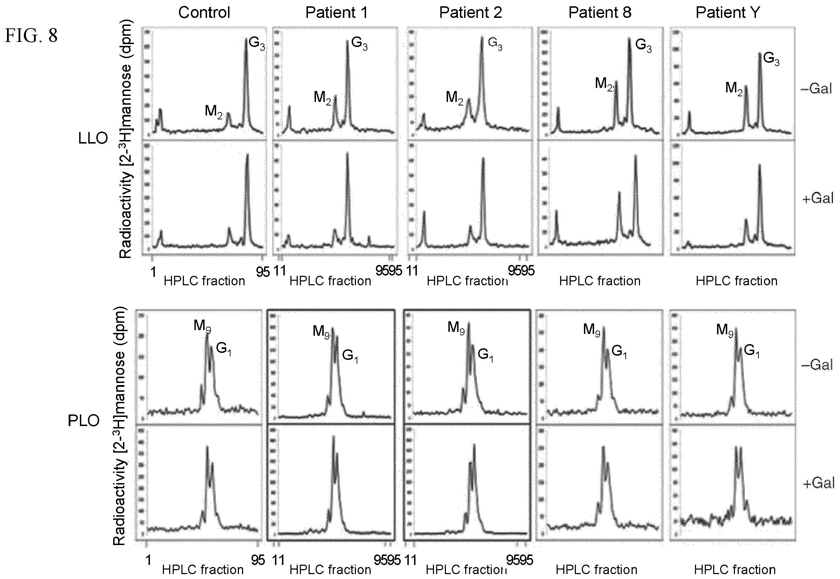

Figure 8 shows a lipid-linked oligosaccharide (LLO) analysis in 4 PGM1 deficient cell lines showing reduced LLO at baseline and improvement following D-gal supplementation.

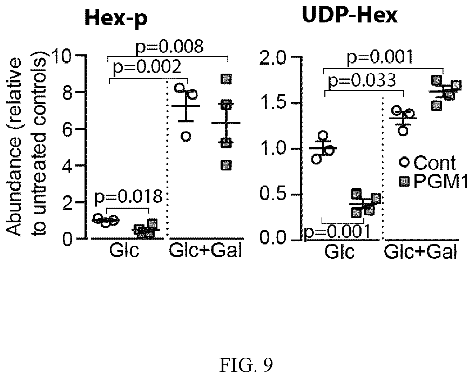

Figure 9 shows changes in concentration and changes in abundance of UDP sugars in patients and controls with and without galactose treatment.

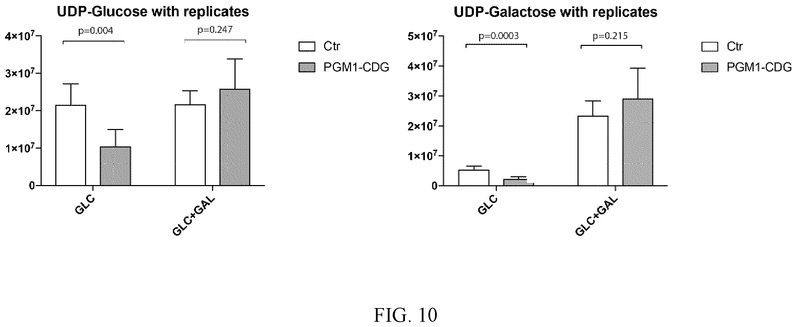

Figure 10 contains graphs showing UDP glucose levels (left graph) and UDP galactose levels (right graph) in PGM1 patient fibroblasts (grey bar) compared to controls (white bar). Glc: regular culture conditions; Glc+gal: galactose added to the media. Both UDP glucose and UDP galactose levels are decreased in PGM1 patient fibroblasts, and significantly increase upon adding galactose to the culture media.

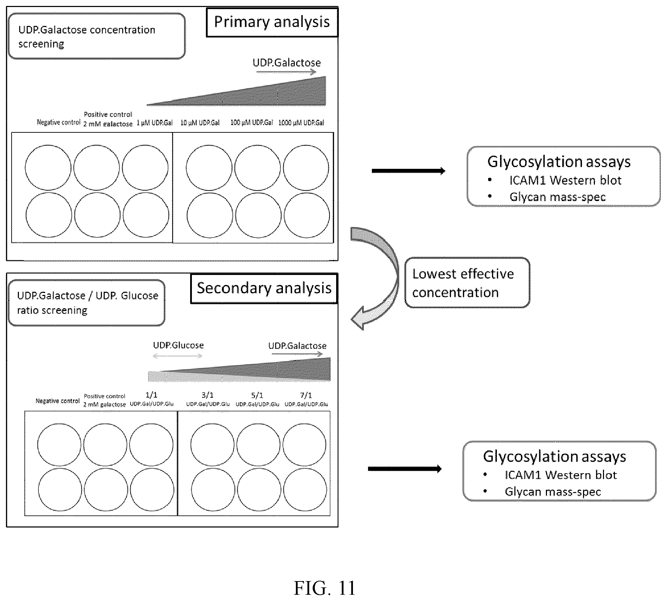

Figure 11 contains a schematic overview of UDP-galactose and UDP-galactose/UDP-glucose concentration screening. Top panel: Primary analysis for assessment of the lowest effective concentration of UDP-galactose for restoration of glycosylation. Lower panel: Secondary analysis for assessment of the optimal UDP-galactose/UDP-glucose ratio for restoration of glycosylation.

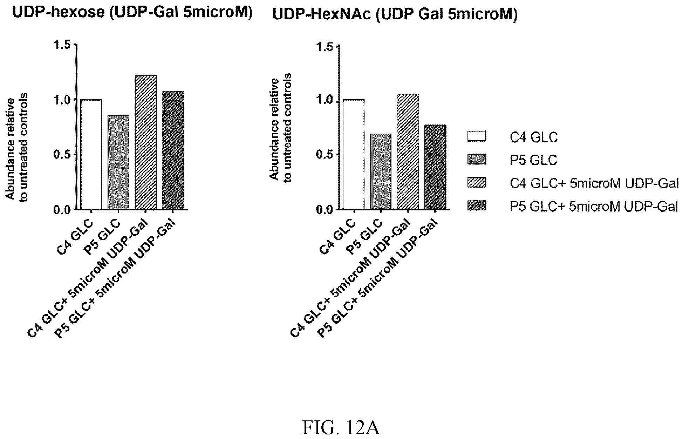

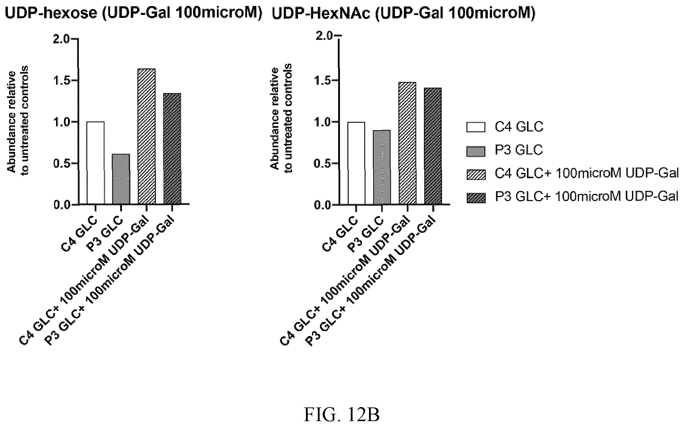

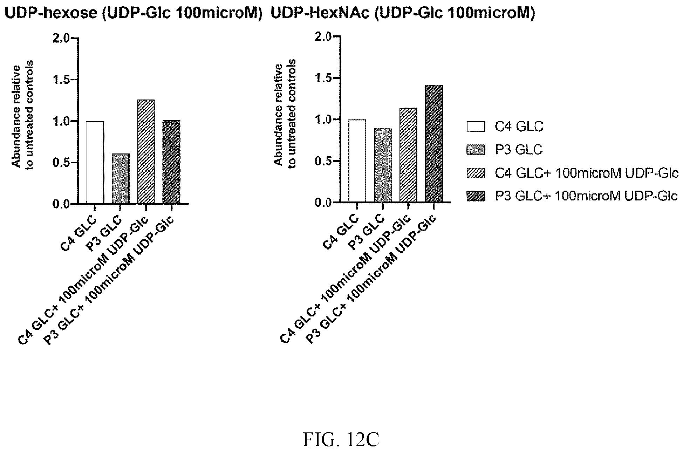

Figures 12A - 12C contain graphs showing a relative abundance of UDP-Hexose or UDP-HexNAc. Figure 12A shows results following treatment with 5 µM UDP-galactose. Figure 12B shows results following treatment with 100 µM UDP-galactose. Figure 12C shows results following treatment with 100 µM UDP-glucose.

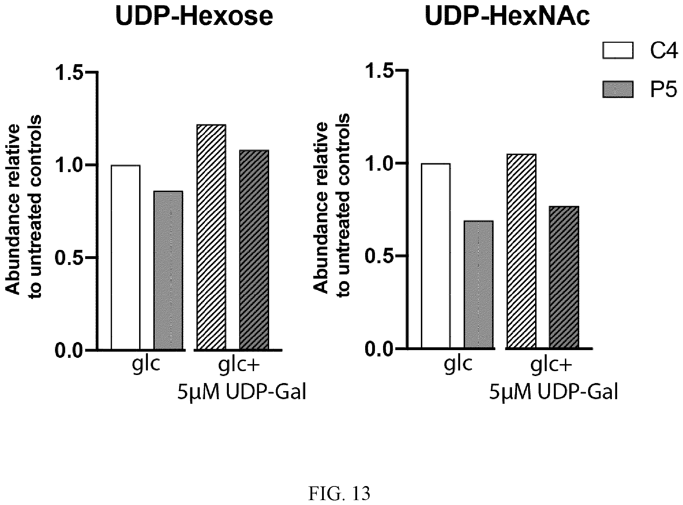

Figure 13 contains graphs showing a relative abundance of UDP-Hexose or UDP-HexNAc following treatment with 5 µM UDP-Galactose (UDP-Gal).

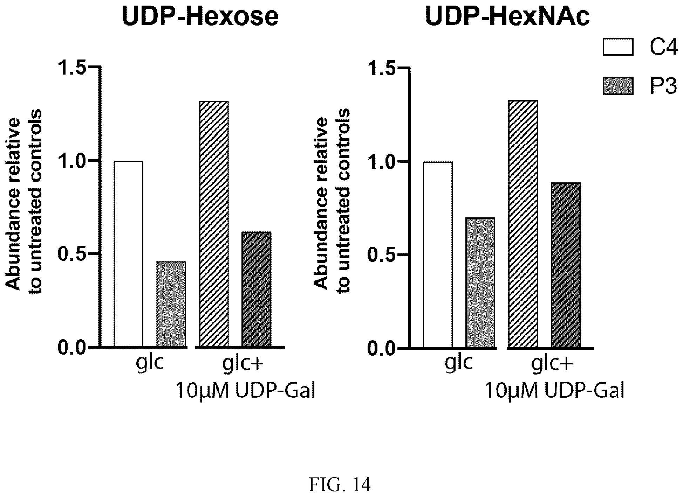

Figure 14 contains graphs showing a relative abundance of UDP-Hexose or UDP-HexNAc following treatment with 10 µM UDP-Galactose (UDP-Gal).

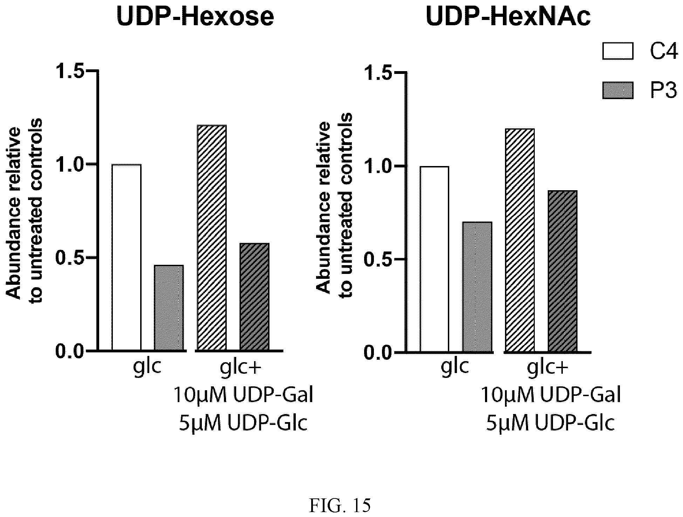

Figure 15 contains graphs showing a relative abundance of UDP-Hexose or UDP-HexNAc following treatment with 10 µM UDP-Galactose (UDP-Gal) and 5 µM UDP-Glucose (UDP-Glc).

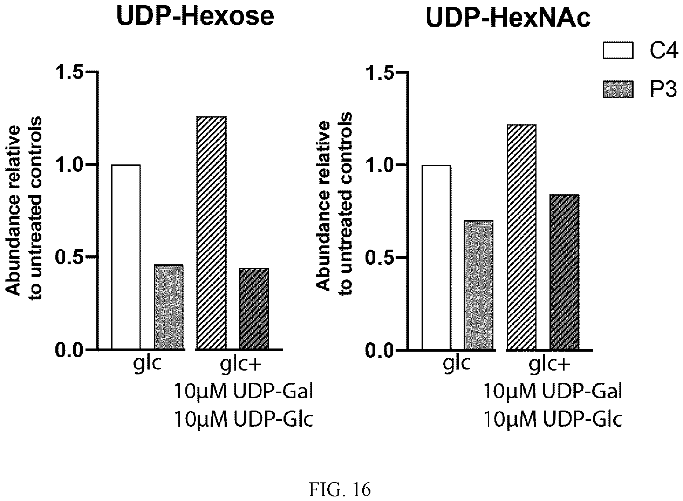

Figure 16 contains graphs showing a relative abundance of UDP-Hexose or UDP-HexNAc following treatment with 10 µM UDP-Galactose (UDP-Gal) and 10 µM UDP-Glucose (UDP-Glc).

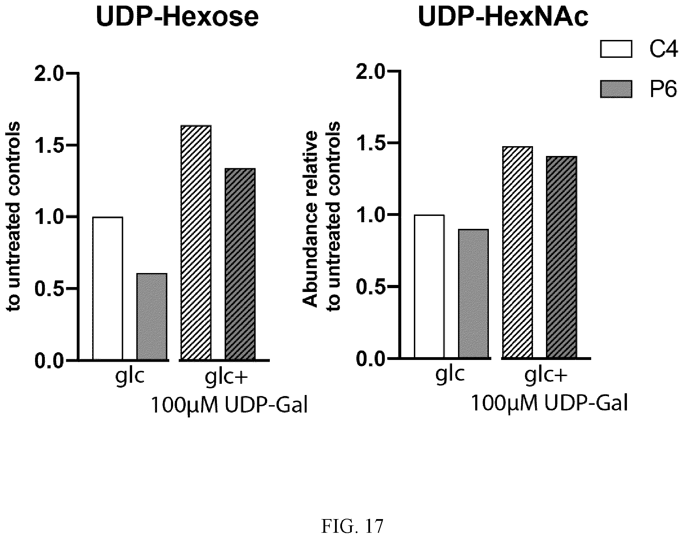

Figure 17 contains graphs showing a relative abundance of UDP-Hexose or UDP-HexNAc following treatment with 100 µM UDP-Galactose (UDP-Gal).

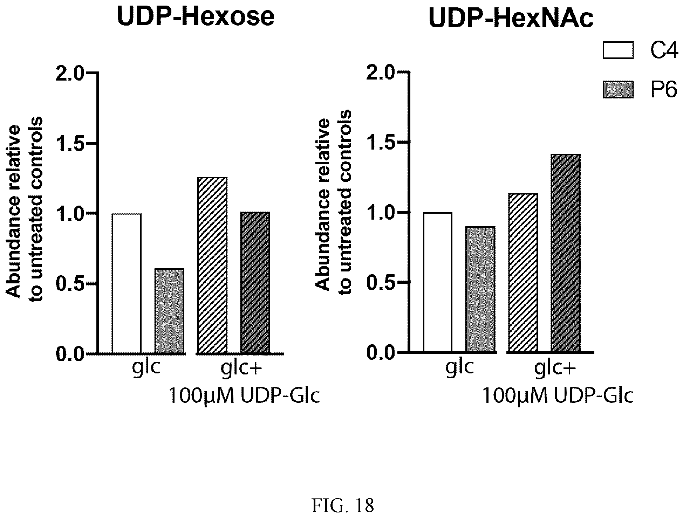

Figure 18 contains graphs showing a relative abundance of UDP-Hexose or UDP-HexNAc following treatment with 100 µM UDP-Glucose (UDP-Glc).

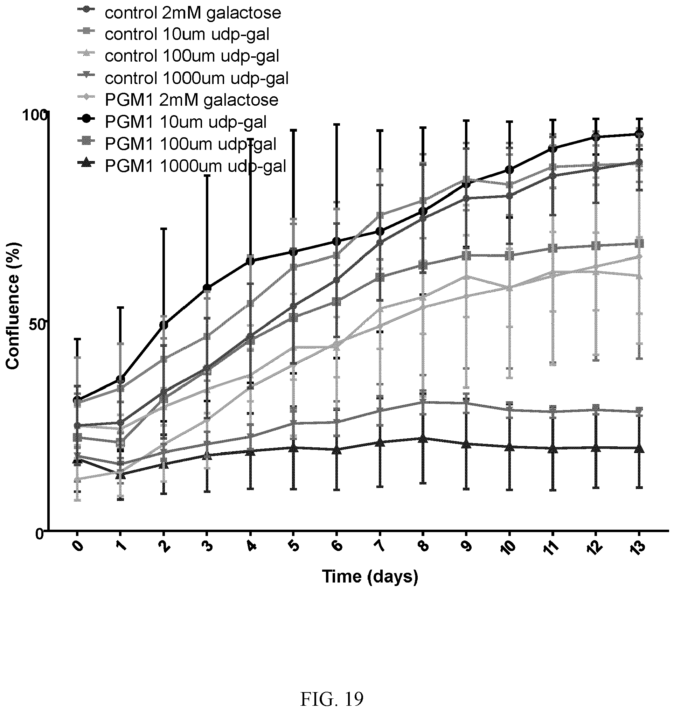

Figure 19 contains a graph showing results from a UDP-galactose cell proliferation assay. Effects of UDP-galactose concentrations on cell proliferation were investigated in healthy control and PGM1-CDG patient derived fibroblast samples. Tested UDP-galactose concentration ranged from 1-1000 µM. Significant inhibitory effects of cell proliferation were observed for 1000 µM UDP-galactose in PGM1-patients cells (linear regression ANCOVA p= 0.0006), but not for 1-100 µM UDP-galactose. Error bars indicate standard error of means.

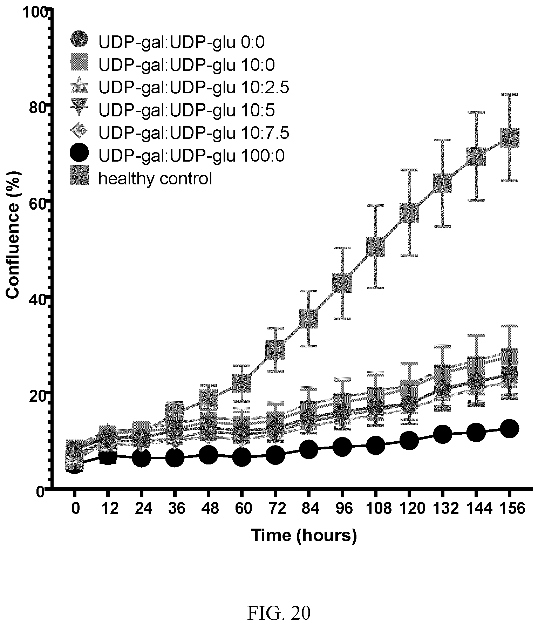

Figure 20 contains a graphs showing results from a combined UDP-galactose: UDP-glucose supplementation cell proliferation assay. Effects of co-supplemented UDP-galactose: UDP-glucose concentrations on cell proliferation were investigated in PGM1-CDG patient derived fibroblast samples. Tested UDP-galactose concentrations were set at 10 µM, and 100 µM, with UDP-glucose levels ranging from 0-7.5 µM. Reduced proliferation growth was observed in the 100 µM UDP-galactose (linear regression ANCOVA p >0.0001), but nor for conditions employing 10 µM UDP-galactose and 0-7.5 µM concentrations of UDP-glucose. Error bars indicate standard error of means.

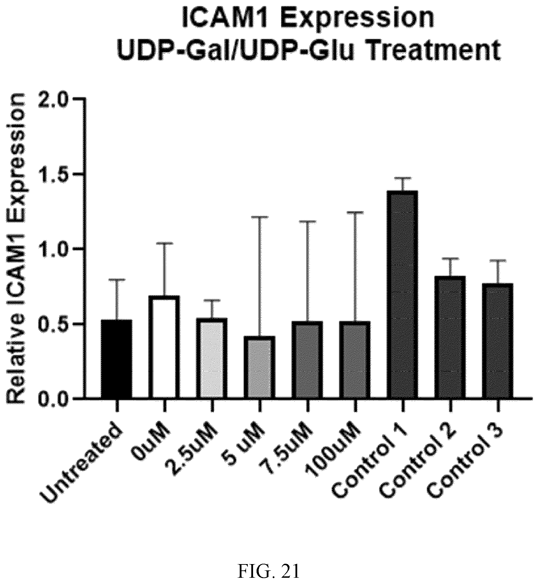

Figure 21 contains a graph of ICAM expression obtained by Western blots obtained from three patient cell lines and controls under different UDP-Gal/UDP-Glu treatment conditions. Treatment conditions include 0: no UDP-glc added, only UDP-gal; 2.5 µM: 10 µM UDP-gal + 2.5 µM UDP-Glc; 5 µM: 10 µM UDP-gal + 5 µM UDP-Glc; 7.5 µM: 10 µM UDP-gal +7.5 µM UDP-Glc; and 10 µM: 10 µM UDP-gal + 10 µM UDP-Glc.

### DETAILED DESCRIPTION

This document provides methods and materials for treating a mammal (e.g., a human) having a CDG (e.g., PGM1-CDG). For example, a composition including one or more (e.g., one, two, three, or more) UDP-sugars can be administered to a mammal having a CDG to treat the mammal. In some cases, a composition including a UDP-galactose and glucose or a derivative thereof (e.g., UDP-glucose) can be administered to a human having PGM1-CDG to treat the human.

Any appropriate mammal having a CDG (e.g., PGM1-CDG) can be treated as described herein. Examples of mammals having a CDG that can be treated by administering one or more UDP-sugars include, without limitation, humans, non-human primates (e.g., monkeys), dogs, cats, horses, cows, pigs, sheep, mice, and rats. For example, a human having PGM1-CDG can be treated by administering one or more UDP-sugars to that human.

When treating a mammal (e.g., a human) having a CDG (e.g., PGM1-CDG) as described herein, the CDG can be any appropriate CDG. In some cases, a CDG can be caused by one or more mutations (e.g., inactivating mutations) in an enzyme involved in glycosylation (e.g., an enzyme that attaches one or more glycans to a polypeptide). An enzyme involved in glycosylation can be involved in any type of glycosylation. Examples of types of glycosylation include, without limitation, N-linked glycosylation, O-linked glycosylation, phosphoserine glycosylation, C-mannosylation, glycosphingolipid and glycosylphosphatidylinositol anchor glycosylation, and multiple glycosylation pathways. Examples of enzymes involved in glycosylation include, without limitation, PGM1, phosphomannomutase 1 (PMM1), phosphomannomutase 2 (PMM2), and mannose-6 phosphate isomerase (MPI). In some cases, one or more inactivating mutations in an enzyme involved in glycosylation can disrupt synthesis of lipid-linked oligosaccharide (LLO) precursors or the transfer of LLO precursors to a polypeptide (e.g., resulting in Type I CDGs). In some cases, one or more inactivating mutations in an enzyme involved in glycosylation can alter trimming and/or processing of a polypeptide-bound oligosaccharide chain (e.g., resulting in Type II CDGs). Typically, a CDG is named based on the enzymatic deficiency causing the CDG. For example, a CDG caused by a PGM1 deficiency is termed PGM1-CDG. Examples of CDGs that can be treated as described herein include, without limitation, PGM1-CDG, PMM2-CDG, and MPI-CDG. In some cases, a CDG, an enzyme involved in glycosylation, and/or CDG nomenclature can be as described elsewhere (see, e.g., Jaeken et al., 2009 Biochim Biophys Acta. 1792:825-826; Ferreira et al., 2018 J Inherit Metab Dis. 41:541-553; and Brasil et al., 2018 Int JMol Sci. 19:E1304). For example, a mammal having PGM1-CDG can be treated by administering one or more UDP-sugars to that mammal.

A CDG can affect any appropriate body part (e.g., tissue, cell type, or organ) of a mammal. Examples of tissues that can be affected by a CDG include, without limitation, nervous tissues, muscles, intestines, heart, liver, endocrine tissues, and skeletal system tissues.

In some cases, methods described herein can include identifying a mammal as having a CDG (e.g., PGM1-CDG). A mammal having a CDG can be identified using any appropriate method. For example, glycosylation status, liver enzyme abnormalities, and/or coagulation abnormalities), hypoglycemia, genetic testing, and/or enzyme activity testing can be used to identify mammals (e.g., humans) having a CDG. In some cases, a glycosylation status of transferrin (Tf) can be used to identify mammals (e.g., humans) having a CDG. Tf glycosylation can be detected using any appropriate methods and/or techniques (e.g., by isoelectric focusing (IEF), or by mass spectrometry such as electrospray ionization-mass spectrometry (ESI-MS). In some cases, altered (e.g., elevated) levels of one or more liver enzymes (e.g., ALT and AST) can be used to identify mammals (e.g., humans) having a CDG. Levels of liver enzymes can be detected using any appropriate methods and/or techniques (e.g., Western blotting techniques). In some cases, altered (e.g., increased or decreased) levels of one or more coagulation factors (e.g., Factor IX, Factor XI, Factor XIII, aPTT, and ATIII) can be used to identify mammals (e.g., humans) having a CDG. Levels of coagulation factors can be detected using any appropriate methods and/or techniques (e.g., enzyme-linked immunosorbent assays (ELISA)). Tf glycosylation, levels of liver enzymes, and/or levels of coagulation factors can be detected in any appropriate sample (e.g., a blood sample such as serum or a cell sample such as fibroblasts) obtained from a mammal (e.g., a mammal having a CDGs). In some cases, identifying a mammal as having a CDG also can include determining a subtype of CDG using, for example, one or more enzyme assays.

A composition including one or more UDP-sugars can include any appropriate UDP-sugar(s). In some cases, a UDP-sugar can be a glycosyl donor in a glycosylation reaction. A UDP-sugar can include any appropriate type of sugar. In some cases, a sugar in a UDP-sugar can be a hexose or a hexose derivative. Examples of hexoses and hexose derivatives that can be used as described herein include, without limitation, galactose (Gal), glucose (Glc), Glucose phosphate (Glc-P), Galactose phosphate (Gal-P), N-acetylgalactosamine (GalNAc), N-acetylglucosamine (GlcNAc), and glucuronic acid (GlcA). In some cases, a sugar in a UDP-sugar can be a pentose or a pentose derivative. Examples of pentoses and pentose derivatives that can be used as described herein include, without limitation, xylose (Xyl), ribose, and ribulose. A sugar in a UDP-sugar can be any appropriate isomer (e.g., a D isomer or an L isomer). A sugar in a UDP-sugar can be any appropriate anomer (e.g., an α anomer or a β anomer). Examples of UDP-sugars that can be used as described herein include, without limitation, UDP-α-D-Glc, UDP-α-D-Gal, UDP-α-D-GalNAc, UDP-α-D-GlcNAc, UDP-α-D-GlcA, and UDP-α-D-Xyl. In some cases, a mammal having a CDG can be treated by administering a composition including a UDP-galactose to that mammal. In some cases, a mammal having a CDG can be treated by administering a composition including a UDP-galactose in the presence of glucose or a derivative thereof (e.g., UDP-glucose) to that mammal. In some cases, a mammal having a CDG can be treated by administering a composition including a UDP-galactose and a UDP-glucose to that mammal.

In some cases, when a mammal having a CDG is treated by administering a composition including a UDP-galactose and a glucose or a derivative thereof (e.g., UDP-glucose) to that mammal, the UDP-galactose and the glucose or a derivative thereof can be administered in any appropriate ratio of UDP-galactose to glucose or a derivative thereof (e.g., UDP-galactose : glucose or UDP-glucose). For example, a composition including a UDP-galactose and a glucose or a derivative thereof (e.g., UDP-glucose) can include UDP-galactose and glucose or a derivative thereof at a ratio of from about 1:1 to about 10:1 (e.g., from about 1:1 to about 10:1, from about 1.3:1 to about 10:1, from about 1.6:1 to about 10:1, from about 2:1 to about 10:1, from about 2.5:1 to about 10:1, from about 3:1 to about 10:1, from about 4:1 to about 10:1, from about 5:1 to about 10:1, from about 6:1 to about 10:1, from about 7:1 to about 10:1, from about 8:1 to about 10:1, from about 1:1 to about 9:1, from about 1:1 to about 8:1, from about 1:1 to about 7:1, from about 1:1 to about 6:1, from about 1:1 to about 5:1, from about 1:1 to about 4:1, from about 1:1 to about 3:1, from about 1:1 to about 2:1, from about 1.3:1 to about 9:1, from about 1.6:1 to about 8:1, from about 2:1 to about 7:1, or from about 2.5:1 to about 6:1). In some cases, a composition including a UDP-galactose and a glucose or a derivative thereof (e.g., UDP-glucose) can include UDP-galactose and UDP-glucose at a ratio of about 3:1.

In some cases, treating a mammal having a CDG (e.g., PGM1-CDG) as described herein (e.g., by administering a composition including one or more UDP-sugars to the mammal) can be effective to reduce the severity of the CDG and/or to reduce one or more symptoms of the CDG. Examples of symptoms of CDGs include, without limitation, cardiomyopathies, endocrinopathies, hepatopathy (e.g., presenting as elevated liver enzymes such as ALT and AST), coagulopathy (e.g., bleeding tendencies and abnormal clotting), hypotonia (low muscle tone), hypoglycemia, developmental delay, esotropia (crossed eyes), seizures, cerebellar hypoplasia, ataxia (poor balance and movement coordination), dysarthria (slurred speech), absent puberty (e.g., absent puberty in females), retinitis pigmentosa (pigment in the retina of the eye), progressive scoliosis (curvature of the spine), and joint contractures. In some cases, symptoms can be as described elsewhere (see, e.g., Ferreira et al., 2018 J Inherit Metab Dis. 41:541-553).

In some cases, treating a mammal having a CDG (e.g., PGM1-CDG) as described herein (e.g., by administering a composition including one or more UDP-sugars to the mammal) can be effective to restore glycosylation in the mammal (e.g., relative to a control sample). Control samples can include, without limitation, samples from normal (e.g., healthy) mammals, cell lines (e.g., cell lines having wild type PGM1 enzyme), samples from the mammal being treated as described herein that were obtained prior to treatment, and samples from the mammal being treated as described herein that were obtained earlier in the course treatment. Any appropriate method can be used to assess glycosylation. In some cases, glycosylation can be assessed by measuring glycosylation of transferrin polypeptides in a sample (e.g., a blood sample such as serum or a cellular sample such as fibroblasts) from a mammal treated as described herein. Examples of methods that can be used to assess glycosylation include, without limitation, Tf glycosylation (e.g., by IEF or by ESI-MS), glycan profiling (glycomics; e.g., by mass spectrometry such as matrix-assisted laser desorption/ionization time-of-flight (MALDI-TOF) MS), and quantification of glycosylated ICAM1 (e.g., by Western blot). In some cases, treating a mammal having a CDG as described herein can be effective to decrease a ratio of a reduced glycosylation form (e.g., A-glyco, mono-glyco, and trisialo-glyco transferrin) over a normal glycosylation form of transferrin (e.g., tetrasialo-glyco transferrin or di-glyco transferrin) in the mammal. For example, treating a mammal having PGM1-CDG by administering a composition including a UDP-galactose and a glucose or a derivative thereof (e.g., UDP-glucose) can be effective to decrease a ratio of A-glyco transferrin over tetrasialo-glyco transferrin in the mammal to from about 0.05 to about 0.45. For example, treating a mammal having PGM1-CDG by administering a composition including a UDP-galactose and a glucose or a derivative thereof (e.g., UDP-glucose) can be effective to decrease a ratio of mono-glyco transferrin over tetrasialo-glyco transferrin in the mammal to from about 0.1 to about 0.7. For example, treating a mammal having PGM1-CDG by administering a composition including a UDP-galactose and a glucose or a derivative thereof (e.g., UDP-glucose) can be effective to decrease a ratio of trisialo-glyco transferrin over tetrasialo-glyco transferrin in the mammal to from about 0.1 to about 0.5.

In some cases, treating a mammal having a CDG (e.g., PGM1-CDG) as described herein (e.g., by administering a composition including one or more UDP-sugars to the mammal) can be effective to decrease a level of one or more liver enzymes (e.g., ALT and AST) in the mammal (e.g., relative to a control sample). Control samples can include, without limitation, samples from normal (e.g., healthy) mammals, cell lines (e.g., cell lines having wild type PGM1 enzyme), samples from the mammal being treated as described herein that were obtained prior to treatment, and samples from the mammal being treated as described herein that were obtained earlier in the course treatment. Levels of liver enzymes can be detected using any appropriate methods and/or techniques (e.g., Western blotting techniques). In some cases, levels of one or more liver enzymes can be assessed in a sample (e.g., a blood sample such as serum or a cellular sample such as fibroblasts) from a mammal treated as described herein. For example, treating a mammal having PGM1-CDG by administering a composition including a UDP-galactose and a glucose or a derivative thereof (e.g., UDP-glucose) can be effective to decrease a level of AST in the mammal to from about 35 U/L to about 1200 U/L (e.g., from about 35 U/L to about 1000 U/L, from about 35 U/L to about 750 U/L, from about 35 U/L to about 500 U/L, from about 35 U/L to about 250 U/L, from about 35 U/L to about 150 U/L, from about 35 U/L to about 100 U/L, from about 35 U/L to about 75 U/L, from about 50 U/L to about 1200 U/L, from about 100 U/L to about 1200 U/L, from about 250 U/L to about 1200 U/L, from about 500 U/L to about 1200 U/L, from about 750 U/L to about 1200 U/L, from about 1000 U/L to about 1200 U/L, from about 100 U/L to about 1000 U/L, from about 250 U/L to about 750 U/L, from about 350 U/L to about 650 U/L, from about 50 U/L to about 100 U/L, from about 100 U/L to about 300 U/L, from about 300 U/L to about 500 U/L, from about 500 U/L to about 700 U/L, or from about 700 U/L to about 900 U/L). For example, treating a mammal having PGM1-CDG by administering a composition including a UDP-galactose and a glucose or a derivative thereof (e.g., UDP-glucose) can be effective to decrease a level of ALT in the mammal to from about 32 U/L to about 50 U/L (e.g., from about 32 U/L to about 40 U/L, from about 32 U/L to about 35 U/L, from about 32 U/L to about 40 U/L, from about 32 U/L to about 35 U/L, from about 35 U/L to about 50 U/L, from about 40 U/L to about 50 U/L, from about 45 U/L to about 50 U/L, from about 35 U/L to about 45 U/L, from about 35 U/L to about 40 U/L, or from about 40 U/L to about 45 U/L).

In some cases, treating a mammal having a CDG (e.g., PGM1-CDG) as described herein (e.g., by administering a composition including one or more UDP-sugars to the mammal) can be effective to alter (e.g., increase or decrease) a level of one or more coagulation factors (e.g., Factor IX, Factor XI, Factor XIII, aPTT, and ATIII) in the mammal (e.g., relative to a control sample). Control samples can include, without limitation, samples from normal (e.g., healthy) mammals, cell lines (e.g., cell lines having wild type PGM1 enzyme), samples from the mammal being treated as described herein that were obtained prior to treatment, and samples from the mammal being treated as described herein that were obtained earlier in the course treatment. Levels of coagulation factors can be detected using any appropriate methods and/or techniques (e.g., viscoelastic methods and chromogenic methods). In some cases, levels of one or more coagulation factors can be assessed in a sample (e.g., a blood sample such as serum or a cellular sample such as fibroblasts) from a mammal treated as described herein. For example, treating a mammal having PGM1-CDG by administering a composition including a UDP-galactose and a glucose or a derivative thereof (e.g., UDP-glucose) can be effective to decrease a level of aPTT in the mammal to from about 21 seconds to about 34 seconds (e.g., from about 21 to about 32, from about 21 to about 30, from about 21 to about 28, from about 21 to about 25, from about 21 to about 23, from about 23 to about 34, from about 25 to about 34, from about 28 to about 34, from about 30 to about 34, from about 32 to about 34, from about 23 to about 32, from about 25 to about 30, from about 26 to about 28, from about 22 to about 28, or from about 26 to about 30 seconds). For example, treating a mammal having PGM1-CDG by administering a composition including a UDP-galactose and a glucose or a derivative thereof (e.g., UDP-glucose) can be effective to increase a level of ATIII in the mammal to from about 45% to about 95% (e.g., from about 45% to about 90%, from about 45% to about 80%, from about 45% to about 75%, from about 45% to about 65%, from about 45% to about 55%, from about 45% to about 50%, from about 50% to about 95%, from about 60% to about 95%, from about 70% to about 95%, from about 75% to about 95%, from about 80% to about 95%, from about 85% to about 95%, from about 90% to about 95%, from about 50% to about 85%, from about 60% to about 70%, from about 45% to about 65%, from about 55% to about 75%, or from about 65% to about 85%). For example, treating a mammal having PGM1-CDG by administering a composition including a UDP-galactose and a glucose or a derivative thereof (e.g., UDP-glucose) can be effective to increase a level of Factor XI in the mammal to from about 35% to about 120% (e.g., from about 35% to about 100%, from about 35% to about 75%, from about 35% to about 50%, from about 35% to about 40%, from about 50% to about 120%, from about 75% to about 120%, from about 100% to about 120%, from about 45% to about 115%, from about 65% to about 100%, from about 45% to about 65%, from about 65% to about 85%, or from about 85% to about 105%). For example, treating a mammal having PGM1-CDG by administering a composition including a UDP-galactose and a glucose or a derivative thereof (e.g., UDP-glucose) can be effective to increase a level of Factor XIII in the mammal to from about 60% to about 120% (e.g., from about 70% to about 120%, from about 80% to about 120%, from about 90% to about 120%, from about 100% to about 120%, from about 60% to about 110%, from about 60% to about 100%, from about 60% to about 90%, from about 60% to about 80%, or from about 75% to about 100%).

In some cases, a composition including one or more UDP-sugars can be formulated into a pharmaceutically acceptable composition for administration to a mammal having a CDG (e.g., PGM1-CDG). For example, one or more UDP-sugars can be formulated together with one or more pharmaceutically acceptable carriers (additives) and/or diluents. Pharmaceutically acceptable carriers, fillers, and vehicles that can be used in a pharmaceutical composition described herein include, without limitation, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, and wool fat.

A composition including one or more UDP-sugars can be designed for oral or parenteral (including subcutaneous, intramuscular, intravenous, and intradermal) administration to a mammal (e.g., a human) having a CDG (e.g., PGM1-CDG). Compositions suitable for oral administration include, without limitation, liquids, tablets, capsules, pills, powders, gels, and granules. Compositions suitable for parenteral administration include, without limitation, aqueous and non-aqueous sterile injection solutions that can contain anti-oxidants, buffers, bacteriostats, and solutes that render the formulation isotonic with the blood of the intended recipient. In some cases, a composition including one or more UDP-sugars can be formulated for oral administration (e.g., as a dietary supplement).

A composition including one or more UDP-sugars can be administered locally or systemically to a mammal (e.g., a human) having a CDG (e.g., PGM1-CDG). For example, a composition including one or more UDP-sugars can be can be administered locally to a mammal (e.g., a human) having a CDG by injection into or near a body part (e.g., tissue, cell type, or organ) of the mammal that is affected by the CDG. For example, a composition including one or more UDP-sugars can be administered systemically by oral administration to a mammal (e.g., a human) having a CDG.

In some cases, a composition including one or more UDP-sugars can one or more additional components. For example, in cases where a composition including one or more UDP-sugars is formulated for oral administration, the composition also can include one or more components to protect UDP-sugars against degradation. For example, in cases where a composition including one or more UDP-sugars is formulated for oral administration, the composition also can include one or more components to protect UDP-sugars against uptake by one or more microorganisms in a microbiome.

A composition including one or more UDP-sugars can be administered to a mammal (e.g., a human) having a CDG (e.g., PGM1-CDG) in any appropriate dose(s). Effective doses can vary depending on the severity of the CDG, the route of administration, the age and general health condition of the subject, excipient usage, the possibility of co-usage with other therapeutic treatments such as use of other agents, and the judgment of the treating physician. An effective amount of a composition including one or more UDP-sugars can be any amount that reduces the severity and/or one or more symptom of a condition being treated (*e.g.,* a CDG such as PGM1-CDG) without producing significant toxicity to the mammal. For example, an effective amount of UDP-galactose can be any amount that results in a blood concentration of galactose of from about 5 µM to about 90 µM (*e.g.,* from about 5 µM to about 80 µM, from about 5 µM to about 70 µM, from about 5 µM to about 60 µM, from about 5 µM to about 50 µM, from about 5 µM to about 40 µM, from about 5 µM to about 30 µM, from about 5 µM to about 25 µM, from about 5 µM to about 20 µM, from about 5 µM to about 15 µM, from about 5 µM to about 10 µM, from about 10 µM to about 90 µM, from about 20 µM to about 90 µM, from about 30 µM to about 90 µM, from about 35 µM to about 90 µM, from about 40 µM to about 90 µM, from about 45 µM to about 90 µM, from about 50 µM to about 90 µM, from about 60 µM to about 90 µM, from about 70 µM to about 90 µM, from about 80 µM to about 90 µM, from about 10 µM to about 40 µM, from about 30 µM to about 50 µM, from about 40 µM to about 80 µM, from about 20 µM to about 30 µM, from about 10 µM to about 30 µM, or from about 20 µM to about 40 µM). For example, an effective amount of glucose or a derivative thereof (e.g., UDP-glucose) can be any amount that results in a blood concentration of glucose of from about 2 µM to about 20 µM (e.g., from about 2 µM to about 18 µM, from about 2 µM to about 15 µM, from about 2 µM to about 12 µM, from about 2 µM to about 10 µM, from about 2 µM to about 7 µM, from about 2 µM to about 5 µM, from about 5 µM to about 20 µM, from about 8 µM to about 20 µM, from about 10 µM to about 20 µM, from about 12 µM to about 20 µM, from about 15 µM to about 20 µM, from about 17 µM to about 20 µM, from about 5 µM to about 15 µM, from about 7 µM to about 12 µM, from about 5 µM to about 10 µM, or from about 10 µM to about 15 µM). The effective amount can remain constant or can be adjusted as a sliding scale or variable dose depending on the mammal's response to treatment. Various factors can influence the actual effective amount used for a particular application. For example, the frequency of administration, duration of treatment, use of multiple treatment agents, route of administration, and severity of the CDG may require an increase or decrease in the actual effective amount administered.

A composition including one or more UDP-sugars can be administered to a mammal (e.g., a human) having a CDG (e.g., PGM1-CDG) in any appropriate frequency. The frequency of administration can be any frequency that reduces the severity of a symptom of the CDG without producing significant toxicity to the mammal. For example, the frequency of administration can be from about once a day to about ten times a day, from about three times a day to about eight times a day, or from about four times a day to about six times a day. The frequency of administration can remain constant or can be variable during the duration of treatment. As with the effective amount, various factors can influence the actual frequency of administration used for a particular application. For example, the effective amount, duration of treatment, use of multiple treatment agents, route of administration, and severity of the CDG may require an increase or decrease in administration frequency.

A composition including one or more UDP-sugars can be administered to a mammal (e.g., a human) having a CDG (e.g., PGM1-CDG) for any appropriate duration. An effective duration for administering a composition including one or more UDP-sugars can be any duration that reduces the severity of a symptom of the CDG without producing significant toxicity to the mammal. For example, the effective duration can vary from several days to several months or years to a lifetime. In some cases, the effective duration for the treatment of a CDG can range in duration from about 10 years to about a lifetime. Multiple factors can influence the actual effective duration used for a particular treatment. For example, an effective duration can vary with the frequency of administration, effective amount, use of multiple treatment agents, route of administration, and severity of the condition being treated.

In certain instances, a course of treatment and the severity of one or more symptoms related to the condition being treated (e.g., a CDG such as PGM1-CDG) can be monitored. Any appropriate method can be used to determine whether or not the severity of a symptom is reduced. For example, the severity of a symptom of a CDG can be assessed using any appropriate methods and/or techniques, and can be assessed at different time points.

The invention will be further described in the following examples, which do not limit the scope of the invention described in the claims.

### EXAMPLES

### Example 1: Oral D-galactose supplementation in PGM1-CDG

### Materials and Methods

### Study design

The study design was an open-label prospective study. Patients with biochemically and genetically confirmed PGM1 deficiency were recruited into the study. Individuals with an existing diagnosis of aldolase B deficiency, galactosemia, hemolytic uremic syndrome, severe anemia, galactose intolerance, or intellectual disability were excluded from the study. Nine patients were enrolled in the study, five females and four males. Patient age ranged from 19 months to 21 years old at the time of enrollment (mean age 9.75 years). Patient data, cDNA mutations and amino acid changes in PGM1, and the residual PGM1 enzyme activity are listed in Table 1. The clinical features at presentation are summarized in Table 2. Informed written consent was obtained before the start of study. A primary end-point was to assess the safety and tolerability of oral D-gal supplementation and to identify physiological biomarkers that are responsive to D-gal supplementation in a heterogeneous genetic background. The effect of D-gal in PGM1-CDG patients was also monitored via glycomics. Secondary endpoints were the restoration of the plasma glycan subfractions, monitored via glycomics (Table 3), and normalization of antithrombin III activity and serum alanine transaminase (ALT) levels.

Additionally, the biological effect of D-galactose was studied *in vitro* in patient skin fibroblasts.

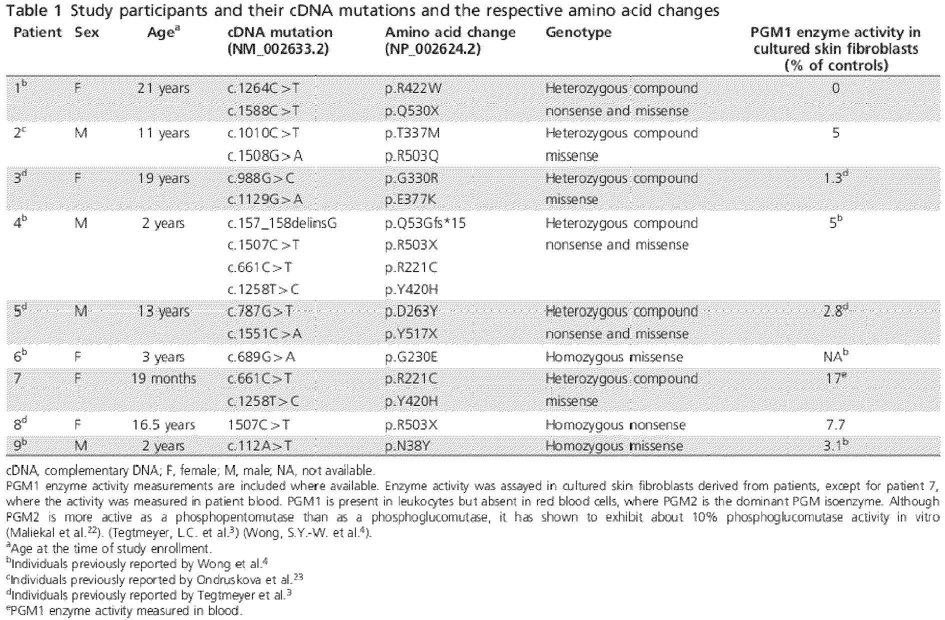

**Table 2. List of all study participants with their respective clinical features in the past clinical course up till they presented at the time of enrollment.**

| Clinical features | | Patients | | | | | | | | |
|---|---|---|---|---|---|---|---|---|---|---|
| | | 1^{#} | ^{∗∗}2% | 3^{$} | 4^{#} | 5^{$} | 6^{#} | 7 | 8^{$} | 9^{#} |
| Congenital malformations | Cleft Palate | + | - | + | + | + | + | - | + | + |
| | Bifid Uvula | - | + | + | + | + | - | - | + | + |
| | Pierre-Robin Sequence | + | - | + | + | + | - | - | + | + |
| | Micrognathia | + | - | - | + | - | - | + | + | - |
| | Others | + | - | - | - | - | - | - | - | - |
| Cardiac | Dilated Cardiomyopathy | + | - | + | - | - | + | - | + | - |
| Muscle | Rhabdomyolysis | + | - | + | - | - | - | - | + | - |
| | Malignant Hyperthermia | - | - | + | - | - | - | - | + | - |
| | Myopathy | + | + | + | + | + | + | + | - | - |
| | Exercise Intolerance | + | + | + | - | - | - | + | - | - |
| | Elevated CK Levels | + | + | + | + | + | + | - | + | + |
| Liver | Elevated Transaminases | + | + | + | + | + | + | + | + | + |
| | Increased Glycogen Storage | - | - | + | - | - | - | N/A | + | N/A |
| | Steatosis | - | - | + | - | + | - | - | + | - |
| Endocrine | Hypothyroidism | - | + | - | - | - | - | - | - | - |
| | IGF1 and IGF3 deficiency | + | + | + | + | + | + | + | + | + |
| | Hyperinsulinism | - | - | - | + | + | - | - | - | - |
| Coagulation | Antithrombin-III deficiency | + | + | + | N/A | - | - | - | + | + |
| | Factor IX deficiency | - | - | - | N/A | - | - | + | + | - |
| | Factor XI deficiency | + | + | + | N/A | + | - | + | + | - |
| Metabolic alterations | Hypoglycemia | + | + | + | + | + | + | + | + | + |
| | Abnormal TIEF | + | + | + | + | + | + | + | + | + |
| Other | Short stature | - | + | + | + | + | + | + | + | + |
| | Recurrent infections | - | + | - | - | + | - | - | - | - |
| | *Learning impairment | - | + | - | + | - | + | - | - | - |
| | Seizures | - | - | - | + | + | - | - | - | - |

| | | | | | | | | | | |
|---|---|---|---|---|---|---|---|---|---|---|
| *Degree of impairment did not fulfill criteria for intellectual disability **Patient had severe obesity $ Individuals previously reported in Tegtmeyer et al., 2014 N Engl J Med 370:533-542 % Individuals previously reported in Ondruskova et al. 2014 Neuro Endocrinol Lett 35: 137-141 # Individuals previously reported in Wong et al., 2016 J Pediatr 175: 130-136.e8 N/A: not available | | | | | | | | | | |

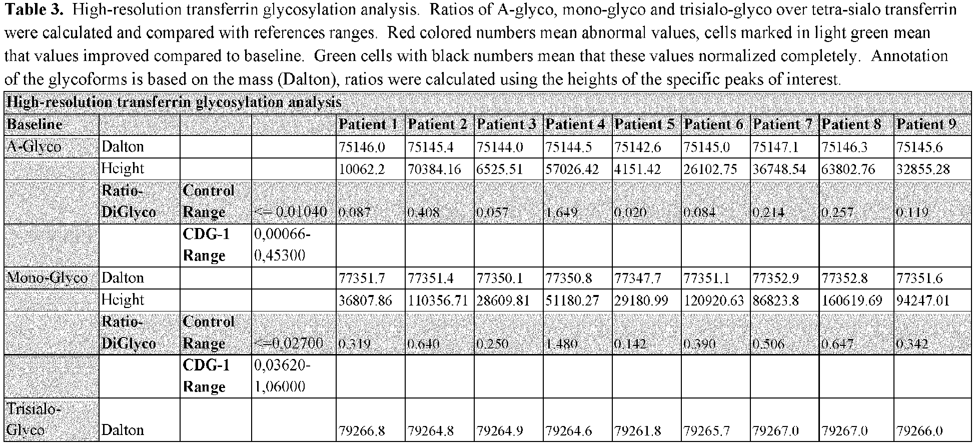

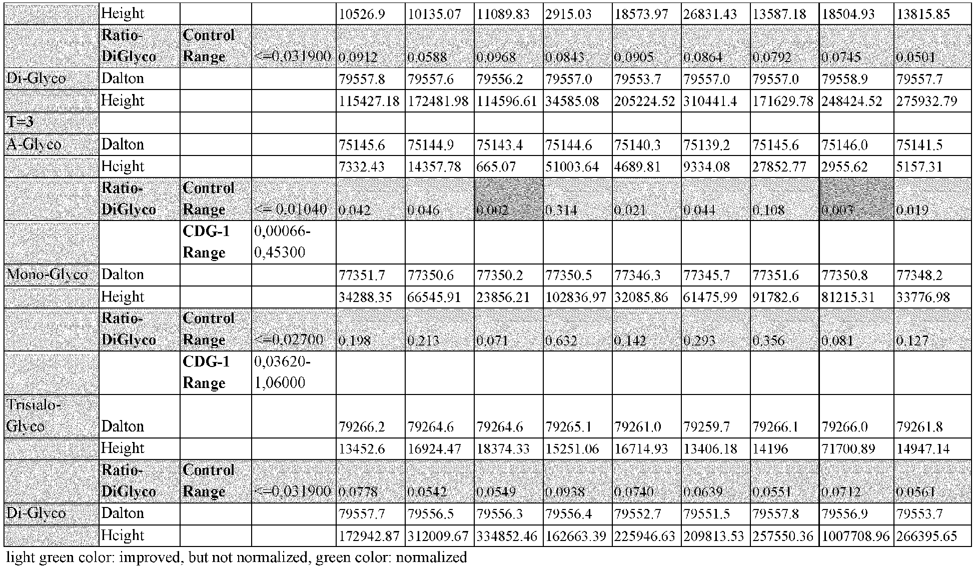

### Three escalating doses of D-gal over 18 weeks

Participants in this pilot study received oral D-gal supplementation, added to the regular diet, over 18 weeks. D-gal (pure and soluble D-galactose powder: D-GALACTOSE or Galaxtra^{™}) was provided as medical food/nutritional supplement. 50 g D-gal equals 5 tablespoons of "powdered sugar". D-gal was added to a meal once a day. It can be added to any food or drink and has a mildly sweet aftertaste.

Natural lactose intake in the diet was monitored, but not controlled in patients, since lactose is a disaccharide (glucose-galactose), not leading to significant increase in blood galactose concentrations.

The D-gal intake was increased over the study period in increments as follows: weeks 0-6 (T0-T1), 0.5 g/kg per day; weeks 6-12 (T1-T2), 1.0 g/kg per day; weeks 12-18 (T2-T3), 1.5 g/kg per day (Figure 2). The intake was increased gradually to reach the recommended maximum daily dose with minimal gastrointestinal irritation (diarrhea, stomachache, nausea) and metabolic side effects (presence of urinary galactitol). The maximum daily oral dose of D-gal any patient received was 50.0 g, an amount that is within the recommended daily maximum intake and demonstrated safe (see, e.g., De Smet et al., 2009 Nephrol Dial Transplant 24:2938-2940).

An oral dose of 0.3g/kg D-gal supplement leads to a blood concentration of D-gal at 0.75mM, 1.0g/kg D-gal dose corresponds to 2.25 mM peak concentration of D-gal in blood, while an oral dose of 1.5g/kg galactose supplement leads to a blood peak concentration of 3.5 mM (Tegtmeyer et al. 2014). The blood concentration of free D-galactose in un-supplemented individuals was below 0.01 mM.

Tolerability and side effects of oral D-gal supplementation, based on studies in blood (glucose, lactic acid, ammonia, galactose-1-phosphate) and urine (galactitol excretion), were monitored at every time point.

Participants were instructed to continue their regular diet. The maximum daily dose of galactose any patient received was 50.0 g, an amount that is within the recommended daily intake (see, e.g., De Smet et al., 2009 Nephrol Dial Transplant 24:2938-2940).

Dietary assessment, clinical evaluation (general physical examination and anthropometric measurements), and laboratory and biochemical studies were completed every 6 weeks (T0, T1, T2 and T3).

Dietary assessment was performed based on a three-days self-report nutritional diary, and diet history obtained by the dietician. The lactose (and galactose) intake was calculated based on the daily dairy intake.

Liver transaminases (aspartate transaminase (AST) and alanine transaminase (ALT)), creatine kinase (CK), commonly assayed glycoproteins (thyroid stimulating hormone (TSH), thyroxine-binding globulin (TBG), and insulin-like growth factor binding protein 3 (IGF3BP)), and coagulation parameters (Factor IX, Factor XI, activated prothrombin time (aPTT), and anti-thrombin III (ATIII)), were measured in blood. Patent 1 had additional Factor XI, and XIII measurements. Serum galactose-1-phosphate and urine galactitol were measured to monitor safety and tolerability of galactose supplementation. Biochemical studies to evaluate changes in glycosylation included isoelectric focusing (IEF) of serum transferrin and glycomic analysis in blood by mass spectrometry.

Electrocardiography/echocardiography and hepatic ultrasonography were conducted at T0 and T3.

### Long-term monitoring

Patient 1 remained on D-gal 1 g/kg/day after the study period as D-gal is the only currently available compassionate treatment for PGM1-CDG. The parameters monitored during the study period, including additional coagulation factor measurements, have been followed as part of routine care (Table 4).

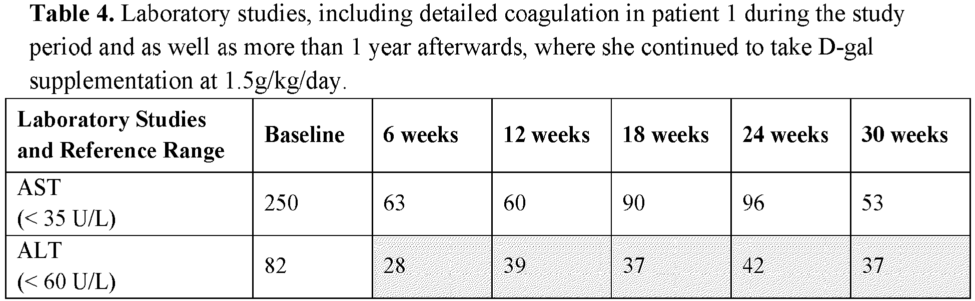

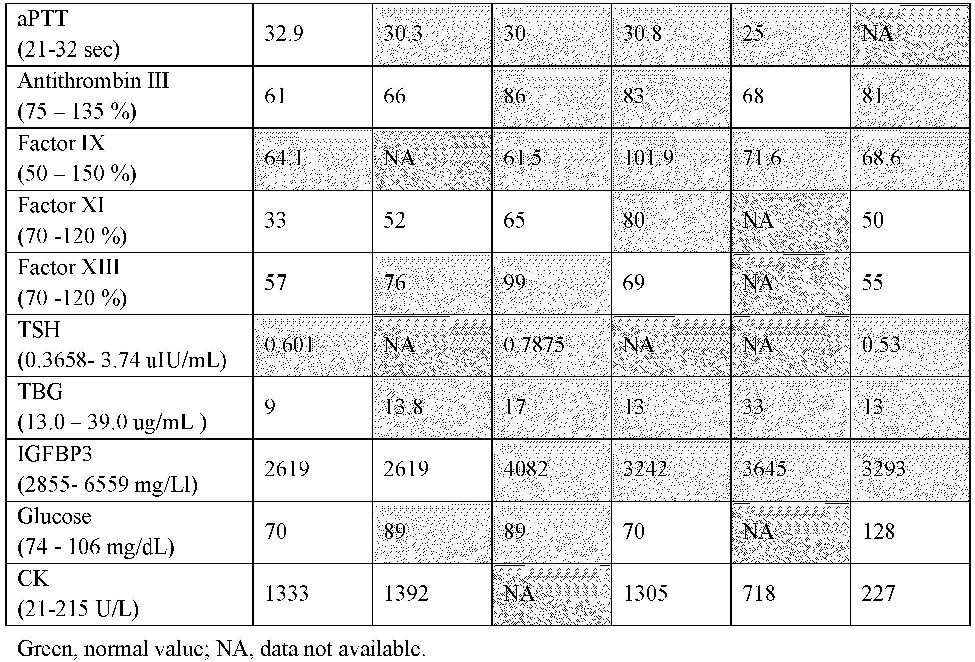

### Glycomics analysis in patient blood at T0 and T3

Plasma (sodium heparin) or serum was used for glycomics analysis according to the procedure as described elsewhere (see, e.g., van Scherpenzeel et al., 2015 Transl Res 166:639-649.e1). Briefly, a 10 µl serum sample was purified using anti-transferrin beads. The eluate was analyzed on a microfluidic 6540 LC-chip-QTOF instrument (Agilent Technologies) using a C8 protein chip. Data analysis was performed using Agilent Mass Hunter Qualitative Analysis Software B.05.00. The Agilent BioConfirm Software was used to deconvolute the charge distribution raw data to reconstructed mass data (see, e.g., Tegtmeyer et al., 2014 N Engl JMed 370:533-542; and van Scherpenzeel et al., 2015 Transl Res 166:639-649.e1).

### Phosphoglucomutase 1 enzyme activity

Skin fibroblast cells were pelleted and washed twice with 1x PBS. Cellular extracts were prepared by lysing the cell pellet in 100 µl homogenization buffer containing 20 mM Hepes, 1 mM DTT, 25 mM KCl, 1 µg/ml leupeptin, and 10 µg/ml antipain. The cell homogenates were centrifuged for 8 minutes at 1,550 × g twice, saving the supernatant each time. The pooled supernatant (200 µl) was stored at -80°C for 24 hours and then centrifuged for 5 minutes at 9,000 × g at 4°C. The cleared lysate was used for protein and enzymatic assay. Total protein was quantified by BCA Protein Assay kit (Thermo Scientific, USA). Phosphoglucomutase 1 enzyme activity was assayed spectrophotometrically at 340 nm as described elsewhere (see, e.g., Van Schaftingen and Jaeken, 1995 FEBS Lett 377:318-20). Briefly, the assay was initiated by the addition of 5 µl of protein extract containing 1-4 mg protein/mL to 100 µl of the assay mixture, which contained 50 mM Hepes (pH 7.1), 10 µg/ml yeast glucose-6-phosphate dehydrogenase, 1 µM glucose-1,6-biphosphate, 0.25 mM NADP, 5 mM MgCl2, and 0.5 mM glucose-1-phosphate. Absorbance was measured by FLUOstar OPTIMA microplate reader (BMG Labtech, Germany) during 10 minutes at 30°C by the reduction of NADP to NADPH.

Diagnostic enzyme analysis was performed in blood in patients when fibroblasts were not available. Similarly, PGM1 enzyme activity in blood was measured with the following modifications: 25 µl of whole blood was added to 250 µl of substrate solution containing 0.25 M Tris (pH 8.0), 0.1% Saponin, 0.4 mM EDTA, 2.0 mM MgCl2, 1.0 mM Glucose-1-phosphate, and 0.6 mM NADP. Sample tubes were incubated in water bath for 30 minutes at 37°C, then 2 µl of blood-substrate mixtures was added to 2 ml of 10 mM of Potassium Phosphate Buffer (pH 7.4). Fluorescence was measured at excitation 360 nm and emission 415 nm.

### Statistics

Quantitative data are presented as the mean ±standard error of the mean. Repeated measures of analysis of variance was used to determine significant differences between pre- and post-D-gal supplement use. p ≤ 0.05 was considered significant.

### In vitro studies characterizing the effect of D-gal supplementation on glycosylation

### Skin fibroblast culture

Skin fibroblasts were derived from skin biopsy via standard clinical and laboratory practices. Cells were cultured in Eagle's minimum essential medium (American Type Culture Collection) supplemented with 10% Fetal Bovine Serum and 1% 100 U Penicillin / 0.1 mg/mL Streptomycin. Skin fibroblasts cells were obtained from four PGM1-CDG patients (Patient 1, 2, 5 and 8) who participated in this study and cell lines were analyzed from two PGM1-CDG patients, used in some of the *in vivo* experiments (Patient Cell-line 2015X and Cell-line2013Y). These patients had been reported elsewhere (see, e.g., Küçükçongar et al., 2015 Genet Couns. 26:87-90; and Pérez et al., 2013 J Inherit Metab Dis. 36:535-42), but were not part of the current clinical observational trial with D-gal. Patient 2015X's mutation (c.1145-222G>T, p.G382Vfs*2) had been reported in two patients (see, e.g., Tegtmeyer et al. 2014 N Engl J Med 370:533-542). Patient 2013Y carries heterozygous compound missense and frameshift mutations (c.871G>A, p.G291R; c.1144+3A>T, p.Arg343fs).

### D-gal supplementation in culture

To investigate whether PGM1 deficiency disrupts the formation of LLOs, a required precursor for the synthesis of nascent N-linked glycoproteins in the ER, LLO and protein-linked oligosaccharide (PLO) analyses were performed in skin fibroblasts of patients 1, 2, 8, cell-line 2013Y and two controls with or without D-gal supplementation (see, e.g., Thiel et al., 2003 J Biol Chem 278:22498-22505).

Fibroblasts were cultured while the culture media was deprived of nutrients and glucose (serum starvation) and metabolically labeled with [2-³H]mannose, as described elsewhere (see, e.g., Thiel et al. 2012 Hum Mutat. 33:485-7). D-gal (Sigma-Aldrich) was added to culture media at concentrations 0, 0.75, 2.0, or 5 mM. The duration of D-gal feeding was 1, 4, 5, or 7 days. Culture media was refreshed every 2 days. For LLO and PLO measurements 10 mM D-gal was added to a serum-deprived culture medium 1 hour prior to the labeling procedure. LLOs and PLOs were extracted by high performance liquid chromatography (HPLC) and analyzed by liquid scintillation counting as described elsewhere (see, e.g., Thiel et al., 2003 J Biol Chem 278:22498-22505).

### PGM1 western blotting

Western blot analysis was performed on the total protein extracted from patient skin fibroblast cells. 25 µg total protein was resolved on 10% Bis-Tris gel at 200 Volts for an hour. Following SDS-PAGE, samples were transferred to a 0.45 µm nitrocellulose membrane at 30 Volts for 2.5 hours on ice. Both running and transfer buffer were supplemented with antioxidant per manufacturer's recommendation (Invitrogen). Membrane was rocked for one hour in SEA BLOCK Blocking Buffer (Thermo Fisher Scientific) at room temperature (RT).

Membrane was rocked overnight with the primary antibodies at 4 °C, followed by six 10-minute washes with 1x Phosphate Buffered Saline (PBS) supplemented with 0.2% Tween 20 (Sigma-Aldrich). Incubation with secondary antibodies at RT for 1 hour was followed by six 10-minute washes with 0.2% Tween-PBS. Membrane was visualized on a Licor Odyssey CLx Infrared Imaging System (LI-COR Biosciences). Signal intensity was quantified by Odyssey software (Version 2.0) and normalized to Beta Actin.

Primary antibody was monoclonal rabbit anti-PGM1 (abcam EPR15240; 1:1400) and monoclonal mouse anti-beta actin (abcam 8226; 1:10000). Secondary antibodies were DyLight 800-conjugated goat anti-rabbit (Thermo Fisher Scientific SA5-35571; 1: 14000) and DyLight 680-conjugated goat anti-mouse (Thermo Fisher Scientific SA5-10082; 1: 18000). Antibodies were diluted in SEA BLOCK Blocking Buffer (Thermo Fisher Scientific).

### ICAM-1 Western Blotting

Intercellular adhesion molecule 1 (ICAM-1), a cell-surface glycoprotein, is a validated hypoglycosylation marker in cultured CDG cells, and its protein expression is diminished in CDG cells. The basal protein expression of ICAM-1, and as well as the effect of galactose supplementation on ICAM-1, was assayed in cultured patient fibroblasts by Western blotting.

Total protein was extracted from 1 million cells and hydrophobic protein was enriched by CelLytic MEM Protein Extraction kit (Sigma-Aldrich). 25 µg total protein or enriched hydrophobic protein was resolved on a 10% Bis-Tris gel. Antioxidant was added to running buffer and transfer buffer per manufacturer's instructions (Life Technologies Novex). Proteins were transferred at 30V for 2 hours on ice. Membrane was rocked for one hour in Membrane Blocking Solution (Thermo Fisher Scientific) at RT and incubated with primary antibodies overnight at 4°C.

Primary antibodies were polyclonal rabbit anti-ICAM-1 (Santa Cruz sc-7891; 1:4000), monoclonal mouse anti-Integrin-β1 (Santa Cruz sc-374429), and monoclonal mouse anti-beta actin (abcam 8226; 1: 10000). Secondary antibodies were DyLight 800-conjugated goat anti-rabbit (Thermo Fisher Scientific SA5-35571; 1: 10000) and DyLight 680-conjugated goat anti-mouse (Thermo Fisher Scientific SA5-10082; 1: 10000 - 1: 18000). Antibodies were diluted in Membrane Blocking Solution.

### Lipid-linked and protein-linked oligosaccharide analysis

Fibroblasts of patients 1, 2, 8, cell-line 2013Y, and two controls were analyzed with or without D-gal supplementation as described elsewhere (see, e.g., Thiel et al., 2003 J Biol Chem 278:22498-22505).

### Nucleotide sugar analysis

Nucleotide sugar analysis for uridine diphosphate (UDP)-galactose (UDP-Gal) and UDP-glucose (UDP-Glc) was performed in 1 million skin fibroblast cells derived from controls; patients 1, 2, and 8; and 2013Y. Either 0.75 or 2.0 mM D-gal was added to the culture medium for 1 day or 4 days prior to harvesting by scraping as described elsewhere (see, e.g., Kochanowski et al., 2006 Anal Biochem 348:243-251).

### Matrix-assisted laser desorption/ionization time-of-flight (MALDI-TOF) mass spectroscopy

Fibroblasts from Patient 2 were used for N- and O-linked glycan analysis with and without D-gal feeding *in vitro.* Upon reaching 100% confluence, the cells were washed twice with PBS and harvested using a cell scraper. The cells were then pelleted and washed with PBS by centrifugation. Fibroblast pellets were lysed in 200 µL PBS, and 200 µg protein from the cell lysate was denatured and precipitated with ~ 2Å volume of 100% propanol. N-linked and O-linked glycans were released from precipitated protein, and free oligosaccharides were purified from the supernatant. N-linked glycans were released from the cell pellets using the PNGase F kit from New England Biolabs (Ipswich, Massachusetts, USA). O-linked glycans were released using β-elimination with sodium borohydride. The released N-linked glycans from cells or deprotonated total cell lysate were purified and desalted by solid phase extraction using a SepPak C18 and carbograph column. O-linked glycans from cells were purified and desalted using an AG 50W-X8 resin cation exchange column. N-linked glycans, O-linked glycans and oligosaccharides were permethylated with sodium hydroxide and iodomethane in dimethyl sulfoxide (DMSO). After permethylation, glycans were extracted with water/chloroform (2:1, vol/vol) in four steps and dried. Samples were then dissolved in 50% methanol, spotted with 11% 2,5-dihydroxylbenzoic acid matrix (1:1 vol/vol), and measured in triplicates and compared to two healthy controls. Measurements were performed by MALDI-TOF using the positive mode on Ultraflex MALDI-TOF/TOF system (BrunkerDaltonics, Billerica, Massachusetts, USA) as described elsewhere (see, e.g., Xia et al., 2013 Anal Biochem 442:178-185).

### Determination of metabolite levels via UPLC-PDA

Levels of nucleotides and sugar-nucleotides were determined. Fibroblast cell pellets (5×10^6 cells per sample) were lysed and extracted in 0.37 ml ice-cold 0.5 M perchloric acid with sonication on ice. Samples were neutralized with 86 µl ice-cold neutralization solution (2.5 M KOH in 1.5 M K₂HPO₄) after 5 minute sonication. To remove precipitated potassium perchlorate neutralized samples were centrifuged at 4°C and 16,400 × g for 10 minutes and filtered with 0.2 µm filters. Nucleotides and sugar-nucleotides were immediately analyzed and separated via ion-pair reverse phased chromatography using an Acquity HSS T3 column (150 mm × 2.1 mm, 1.7 µm, Waters) connected to an Acquity H-class UPLC system. The column temperature was set to 40°C and the column was equilibrated with solvent A (50 mM potassium phosphate buffer, 8 mM tetrabutylammonium hydrogensulfate, pH 6.5) at a flow rate of 0.45 ml min⁻¹. Autosampler temperature was maintained at 6°C. The elution gradient was as follows: After 2.6 minutes 0% B (30 % acetonitrile in 70 % solvent A) to 17 minutes 77% B, hold for 1 minute at 77% B, followed by return to 0% B and conditioning of the column to initial conditions for 10 minutes. Nucleotides were detected by Acquity PDA detector (Waters, 260 nm) and quantified using ultrapure standards (Sigma). Data acquisition and processing was performed with the Empower3 software suite (Waters).

Nucleotide sugars were measured with serum starvation during 18 hours of culturing and without of serum starvation and applying normal media. Nucleotide sugar measurements were done after 1 and 4 days of culturing in the absence or presence of D-gal in the culture medium in the concentration of 0.75 mM and 2.0 mM (Table 5).

### Results

### Escalating dosing of D-gal up to 50 g/day is safe and tolerated

Of the nine patients, eight patients were compliant with daily oral D-gal supplementation. Protocol violations were reported in two patients (Supplementary Data). Patient 8 was non-compliant. She regularly took lactose for more than two years before the study, but her D-gal intake during the study was highly variable. Because patient 8 has not been compliant and her laboratory and clinical studies were incomplete, we excluded her from clinical analysis. Her glycomics analysis was evaluated, however, since her serum was collected throughout the study.

Dietary evaluation reported variable daily dairy intake. No patients consumed more than 0.4 g/kg/day dietary lactose (equivalent to 0.2 g/kg/day D-gal).

Adverse events were monitored weekly. Aside from gastroenteritis in patient 2, no clinical or metabolic adverse events were reported to be associated with incremental increase in D-gal intake. No serious adverse events were reported.

Safety parameters were normal and remained stable during the trial, with no patients experiencing increased galactitol excretion in urine. Galactose-1-phosphate levels were successfully monitored in patients 1, 2, 3, 5, 7, and 9, and no abnormalities were found at T2 and T3. No structural cardiac or liver changes were noted at study endpoint.

### Liver function: improvement in AST and normalization of ALT

Baseline AST of all patients was 3- to 30-fold higher than the upper limit of normal (Figure 3A). AST moderately declined at 6 weeks, and at 12 weeks, AST declined by 23% to more than 10-fold. With the exception of patient 2, AST remained relatively stable, and while it did not normalize at 18 weeks, it fell below baseline. Patient 2 developed an intercurrent illness and discontinued galactose supplementation for 2 weeks between the 12- and 18-week time point. His AST spiked upwards, almost reaching baseline at 18 weeks.

Unlike AST, ALT normalized in several patients (Figure 3B). Baseline ALT was abnormal in 5 patients (patients 1, 2, 4, 5, and 7); the rest exhibited normal ALT throughout the study. Among the 5 patients with abnormal baseline ALT, patients 1, 2, 4, and 7 normalized at 6 or 12 weeks. Except for patient 2, ALT remained normalized and stable at 12 and 18 weeks. While ALT in patient 2 normalized at 6 and 12 weeks, at 18 weeks it spiked upwards and exceeded baseline by 18%. Patient 2 had an intercurrent illness (see above). Patient 5's ALT decreased by 43% at 6 weeks and remained stable at 12 and 18 weeks, at 3% above the upper limit of normal.

ALT levels were found to be significantly decreased from T0 to T2 during the trial (p = 0.05). A dosage of 1.5 g/kg/day in the last 6 weeks of the trial did not lead to significant improvement of the laboratory parameters compared to those at 1 g/kg/day.

### Improvement and normalization of coagulation parameters

Anticoagulation data was available for patients 1, 2, 3, 5, 6, and 9 (Figure 3C). Only patient 9 had a normal baseline. ATIII normalized in patients 1 and 2 at 12 weeks. Patient 1 remained within normal range at 18 weeks. While patient 2's ATIII dropped 30% following illness and cessation of galactose intake, ATIII was maintained at twofold higher than baseline at 18 weeks. Patients 3 and 6 improved by 25% to 50% at 18 weeks, but did not normalize. Patient 5 transiently normalized at 6 weeks, but fell 8% below the lower limit of normal at 12 and 18 weeks.

With the inclusion of all patient data for ATIII there was a significant difference between T0 and T3 data point for ATIII (P =0.020).

The aPTT baseline was normal in patients 2, 3, 5, 6, and remained stable during the study (Figure 3D). aPTT baseline was abnormal in patients 1 and 7 and normalized at 6 and 12 weeks, respectively.

Factor IX data was available for patients 1, 2, 5, 6, and 9, and the baseline was normal (Figure 3E). Factor IX levels remained normal following 18 weeks of galactose supplementation.

Other coagulation parameters, Factor XI and Factor XIII, normalized in patient 1 after 12-18 weeks of galactose supplementation (Figure 3F, Figure 4).

### Improvement in glycoproteins TSH, TBG, and IGFBP-3

TSH data is available for all patients except patient 7 (Figure 3G). Patients 2 and 5 had abnormal baseline. Patient 2 normalized at 18 weeks while patient 5 normalized at 6 weeks and remained stable at 12 and 18 weeks. TBG data is available for patients 1, 2, 5, 6, and 9 (Figure 3H). All patients had abnormal baseline, except for patient 5. Patients 1 and 2 normalized at 6 weeks and remained stable at 12 and 18 weeks. Patient 6 improved by 30% at 12 weeks, only 8% below the lower limit of normal. Patient 9's baseline was twofold the upper limit of normal. Although his TBG level declined by 12% at 18 weeks, it was still 80% higher than the upper limit of normal.

IGFBP-3 data is available for patients 1, 2, 3, 5, and 6 (Figure 3I). Except for patient 5, the baselines were all abnormal, ranging from 8% to 60% below the lower limit of normal. Patients 1 and 2 normalized at 12 weeks and remained stable at 18 weeks. The 12-week time point data was missing for patient 3; nevertheless it normalized at 18 weeks. Patient 6 showed slight improvement at 12 weeks but remained about 50% below the lower limit of normal (Figure 4).

### Fluctuating and abnormal levels of serum creatine kinase and glucose

Creatine kinase and glucose levels fluctuated and remained abnormal during the study. Despite severe elevation of creatine kinase, none of the patients experienced clinical rhabdomyolysis while on galactose supplementation. The frequency of hypoglycemic episodes decreased significantly in four patients. Patients 1 and 6 had recurrent hypoglycemic episodes upon fasting. Patient 5 remained on diazoxide treatment due to hyperinsulinism.

### Improvement of serum transferrin glycosylation in 8 patients

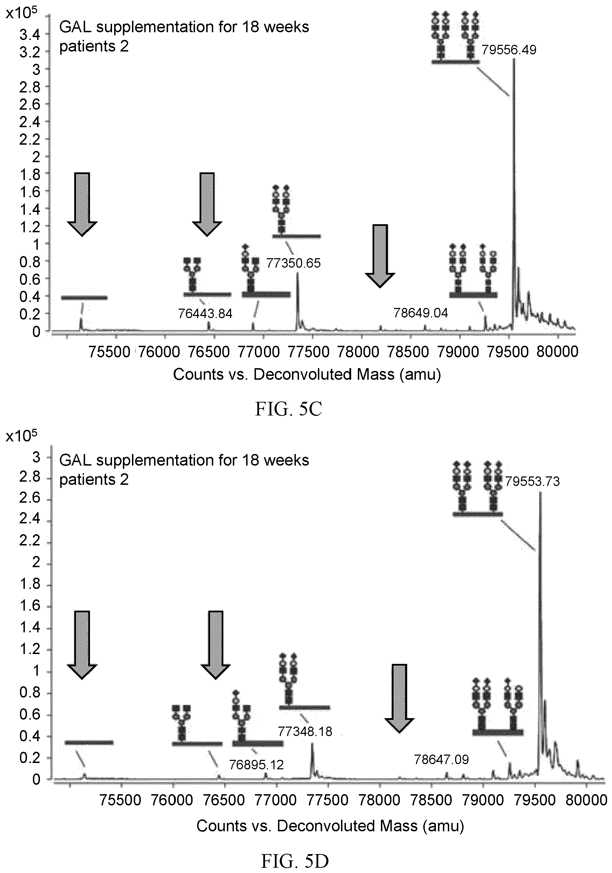

Isoelectric focusing (IEF) and high-resolution mass spectrometry and of intact transferrin confirmed the characteristic mixed type I/II pattern with increased fractions of both truncated glycans and lack of whole glycans in all patients' sera. Clear improvement of transferrin glycosylation was seen in 8 patients, with patterns shifting from a type 2 pattern to a mild type 1 pattern, while no change was observed in patient 5. None of the patients showed a normalized pattern at T3. Figure 5 shows a severely abnormal profile (patient 2) and a mildly abnormal profile (patient 9) at baseline; the improvement in transferrin glycosylation following 18 weeks of galactose supplementation is clear in both patients. Table 3 lists the peak abundances of A-glyco, di-, and tri- and tetrasialo glycoforms and the ratios over the main tetrasialo transferrin glycoform. The A-glyco and di-sialo glycoforms clearly decreased upon galactose treatment, while the tri-sialo glycoform slightly increased in all of the patients. The most pronounced improvement was measured in patient 8. Except for the A-glyco fraction, which completely normalized in patients 3 and 8, none of the other glycan abnormalities fully recovered upon galactose supplements after 18 weeks of treatment (Table 3, Figure 5, and Figure 6).

The most abundant peaks in the transferrin mass spectrometry profiles were annotated, and ratios were calculated for the nonglycosylated (A-Glyco), monoglycosylated (Mono-Glyco), and trisialo- (Trisialo-Glyco) transferrin over the normal transferrin peak (tetrasialo; Di-Glyco). Ratios were compared in patient samples before D-gal supplementation and 18 weeks after starting oral supplements according to protocol. An example is shown in Figure 5 for a severely (patient 2) and a mildly (patient 9) abnormal profile. A-Glyco- and Mono-Glyco ratios were abnormal in all patients. All patients except patient 5 showed significant improvement on D-gal supplementation (Figure 6). The most pronounced improvement was measured in patient 4. In patients 3 and 8, the A-Glyco peak normalized (Table 3, Figure 6).

### Long-term monitoring of patient 1

Patient 1 remained on galactose therapy at 1 g/kg/day for an additional 12 months after the study ended. The parameters that improved and normalized during the study period, including ALT, aPTT, ATIII, TSH, and TBG, remained stable (Table 4). However, Factor XI and Factor XIII returned to before-treatment levels. Surprisingly, creatine kinase, which remained high during the study (five-fold the upper limit of normal), decreased by almost 80% over the course of one year, falling to the near upper limit of normal.

### In vitro studies in PGM1-CDG skin fibroblasts

PGM1 protein expression was moderately reduced in patient 2, who carries heterozygous missense mutations (T337M and R503Q). No protein was visible in patient 1 (R422W and Q530X) and cell-line 2015X (G382Vfs*2) (Figure 7A).

ICAM-1 protein expression was markedly diminished in all four patients, by 5- to 10-fold comparing to two healthy controls (Figure 7A). While galactose supplementation had no effect on ICAM-1 expression in healthy cells (Figure 7A), an increase in ICAM-1 up to 2-fold was observed in patients 1, 2, and cell-line 2015X in a dosage-dependent manner, the highest fold-difference associated with 2 and 5mM galactose supplementation. Furthermore, the initial improvement was observed on day 5 and remained stable on day 7 (Figure 7B). No improvement in ICAM-1 expression was observed in patient 5 across all doses of galactose supplementation (Figure 7C).

Glycomics showed that both asialylated and monosialylated biantennary N-glycan species were elevated (>2 STD from control mean) in N-glycan profiles of total glycoprotein isolated from the cultured skin fibroblasts of patient 2. Following galactose supplementation, asialylated biantennary glycan (Hexose₅HexNAc₄) at m/z 2070.06 was reduced to normal level. Monosialylated glycans (sial1hexose₅HexNAc₃) at m/z 2605+2431, while far from normalized, showed a slight reduction (Table 6).

**Table 6. N-Glycan by mass spectrometry in untreated skin fibroblasts of Patient 2 (column I) and treated with 4 days of 0.75mM galactose following 18 hours of serum starvation (column II).**

| | | | **I. Untreated** | | **II. 0.75 mM Galactose** | |
|---|---|---|---|---|---|---|
| **m/z** | **Predicted components** | **Control range (n=10)** | **% Total glycan** | **Level** | **% Total glycan** | **Level** |
| 2070.06 | Hexose5HexNAc4 | <3.88% | 4.12% | High | 3.65% | **Normal** |
| 2605 | Fuc1Sial1Hexose5HexNAc4 | <4.46% | 7.85% | High | 6.15% | High |
| 2431 | Sial1Hexose5HexNAc4 | <3.90% | 3.23% | **Normal** | 2.23% | **Normal** |
| 2605+2431 | monosialo biantennary glycans | <8.36% | 11.08% | High | 8.38% | High |

Unexpectedly, altered O-glycosylation was detected in patient 2's cultured skin fibroblasts, with decreased disialyl core 1 glycans at m/z 1256 and increased disialyl core 2 at 1706 m/z. These alterations improved but did not completely normalize with galactose supplementation. Interestingly, galactose supplementation increased monosialo core 1 at m/z 895 both in control and PGM1-CDG cells (Table 7).

**Table 7. O-Glycan by mass spectrometry in untreated skin fibroblasts of Patient 2 (column I) and treated with 4 days of 0.75mM galactose following 18 hours of serum starvation (column II).**

| **I. Untreated** | | | **II. 0.75 mM Galactose** | | | |
|---|---|---|---|---|---|---|
| **m/z** | **Predicted components** | **Control range (n=10)** | **% Total glycan** | **Level** | **% Total glycan** | **Level** |
| 1256 | core1, disialyl T | 55.12% | 8.60% | Low | 11.22% | Low |
| 1706 | core2, disalyl, sial2hexose2hexNAc2 | 14% | 42.37% | High | 35.12% | High |

Improvement of glycomics results in fibroblasts on galactose. Interestingly, before *in vitro* galactose treatment the sialylation of N-glycans was found to be reduced, with increased hyposialylated subspecies. Galactose treatment improved sialylation. In addition to their synthesis, scavenge or secretion could also affect plasma glycoprotein glycosylation. Study of cellular glycoproteins indicates that hyposialylation in PGM1 deficiency is likely a deficiency in glycan synthesis, rather than a secondary defect on scavenge or secretion. The Km of sialyltransferase for N-linked glycosylation was estimated at 6 µM. It is therefore surprising that 0.75 mM galactose did not fully normalize the N-glycan profile in PGM1-deficient patients if galactose corrects nucleotide sugar deficiency directly. As *ST6Gal1,* encoding the major sialyltransferase for N-linked glycosylation is a highly regulated gene, it is possible that the slow and partial correction of N-linked glycan sialylation is related to improved *ST6GalI* gene expression rather than a direct effect on the CMP-sialic acid level. No change of GlcNAcylation was detected in either N-linked or O-linked protein glycosylation profiles after galactose treatment in fibroblasts measured by glycomics after serum starvation in patient 2.

The level of full-length LLO (Glc₃Man₉GlcNAc₂-PP-Dol, G3) or sugar moieties bound to newly synthesized PLO (mainly Glc₁Man₉ and Man₉), remained fairly unchanged in control cells (Figure 8, left column), indicating a high degree of metabolic fitness in these cells. In contrast, cells from all four patients showed a large amount of shortened LLO (Man₉GlcNAc₂-PP-dolichol) (Figure 8, LLO top row). In contrast, the PLO profile was indistinguishable from the control cells (Figure 8, PLO top row). Galactose supplementation led to the reduction of shortened LLO (Man₉GlcNAc₂-PP-dolichol) in patients 1, 2, and cell-line 2013Y, resulting in a LLO profile similar to control. No improvement was observed in patient 8 (Figure 8, LLO bottom row). In none of the patients galactose supplementation had an effect on the PLO profile (Figure 8, PLO bottom row).

Because the synthesis of the sugar donor substrate, dolichylphosphate- glucose (Dol-P-Glc), requires UDP-Glc, we hypothesized that PGM1 deficiency would disrupt normal glucose and galactose metabolism, leading to a disbalance of available nucleotide sugars for Dol-P-Glc synthesis and resulting in the observed accumulation of shortened LLO (Man₉GlcNAc₂-PP-dolichol). In patient cell lines 1, 2, 8, and cell-line 2013Y, we found increased levels of UDP-Glc (72.2% and 49.3% of control, 141.3% and 90.5% of control, 60.9% and 23.0% of control, and 142.1% and 87.7% of control, respectively) and in a lesser degree UDP-Gal (29.6% and 23.4% of control, 84.4% 69.7% of control, 9.1% and -22% of control, 48.3% and 32.7% of control, respectively), in the presence of glucose in the medium. D-Gal supplementation increased the levels of both UDP-Glc and UDP-Gal. The same effect in control cells occurred on the first day of D-gal supplements but normalized at 4 days. In all patient cell lines both UDP-Glc and UDP-Gal remained significantly increased on 4 days of D-gal supplementation. Nucleotide sugar ratios UDP-Glc/UDP-Gal in controls were at a mean of 1.2, both with or without D-gal, while in patient cell lines at mean 1.63, they were similar to that on D-gal (1.56); suggesting that adding D-gal didn't restore the nucleotide sugar ratio (Table 5).

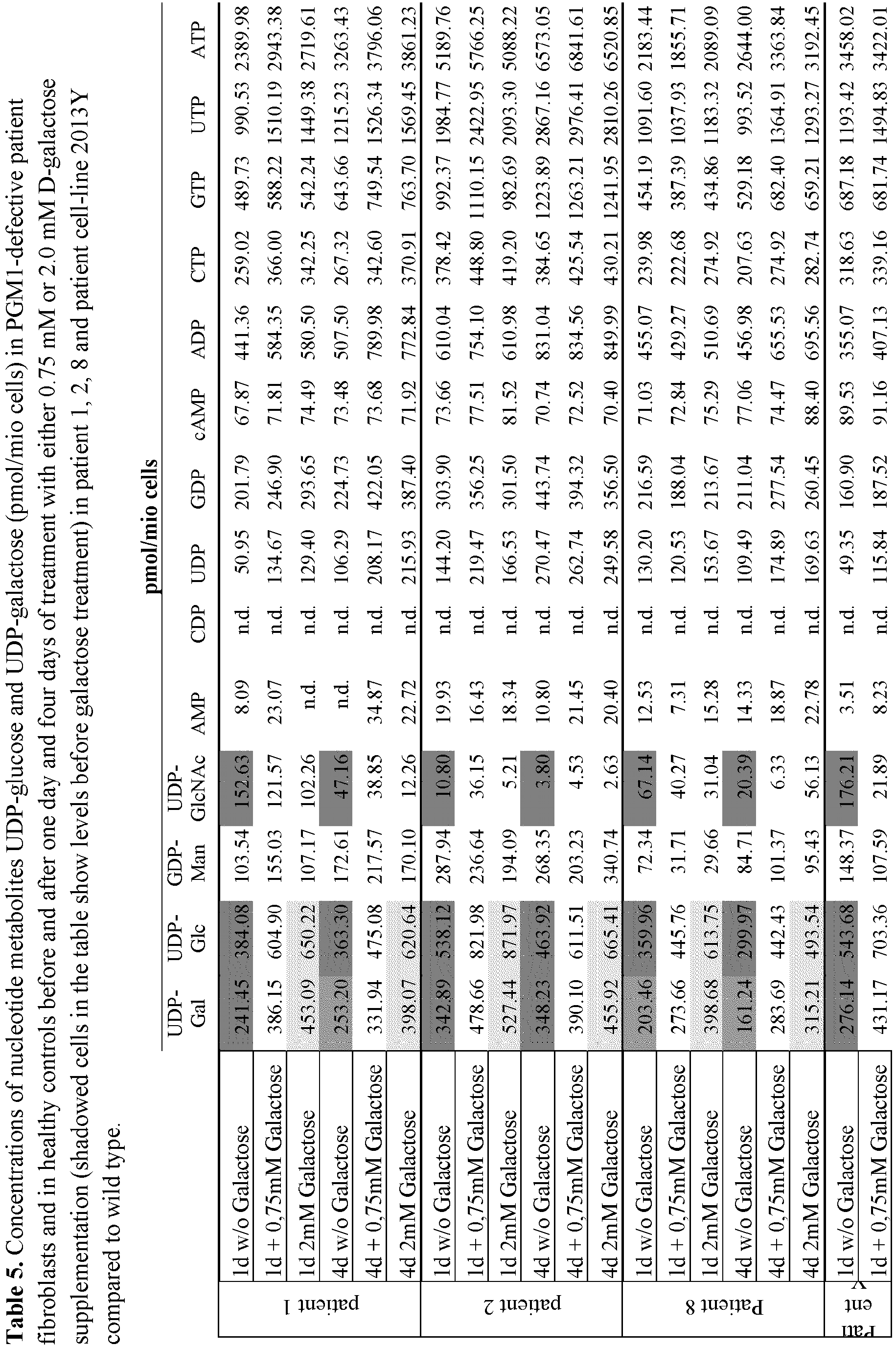

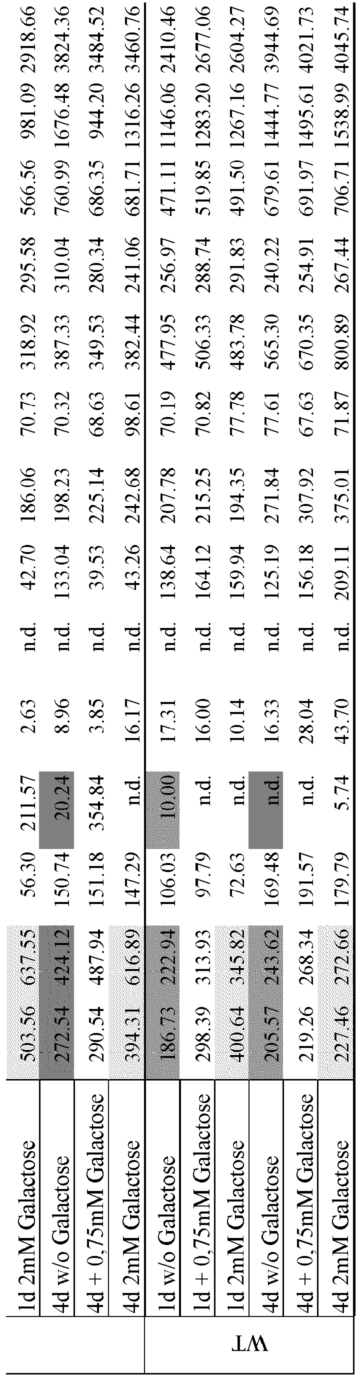

### Conclusions

These results demonstrate D-gal can be orally administered to patients with PGM1-CDG to achieve quick and significant clinical laboratory and metabolic improvements in the patients.

### Example 2: Correcting the PGM1-CDG phenotype

### Methods

To probe the biochemical basis to galactose treatment, a tracer based metabolomics was employed to simultaneously track the activity of multiple pathways. Control and PGM1-CDG fibroblasts were cultured with 13C tagged glucose, with and without galactose. The 13C tracer was then incorporated into connected pathways after which, the cells were harvested to enable detection of metabolites by the mass-spectrometry.

### Results

Supplementing PGM1-CDG fibroblasts with galactose restored depleted levels of the activated sugars UDP-glucose and UDP-galactose, and restarted stalled glycosylation (Figure 9). Glucose is utilized differently in the presence of galactose.

Both UDP glucose and UDP galactose levels were decreased in PGM1 patient fibroblasts, and both UDP glucose and UDP galactose levels were significantly increased when the culture media was supplemented with galactose (Figure 10).

### Conclusions

These results demonstrate that a PGM1-CDG patient can be treated by administering UDP-glucose and UDP-galactose.

### Example 3: UDP-galactose and UDP-glucose concentration screening for restoration of glycosylation in PGMI-CDG fibroblast cells

### Methods

Supplementation assays are performed in PGM1-CDG patient derived fibroblasts from a patient previously enrolled in galactose supplementation protocols as described elsewhere (see, e.g., Wong et al., 2017 Genetics in medicine 19:1226-1235). Cells will be cultured in standard DMEM medium supplemented with glucose (1g/L) with L-glutamine), 10% FCS, and 1X Pen Strep. Cells are seeded at 1500 cells/cm² in 150µL of culture media/cm². Assays are performed in a 6-well microtiter plate containing 2 mL total volume for 9.4 cm growth area. 12 hours after seeding, medium is replaced with standard DMEM medium containing 1 µM, 10 µM, 100 µM, or 1000 µM UDP-galactose. A negative control condition omitting UDP-galactose is included. An additional condition supplemented with 2 mM galactose is included as positive control. UDP-galactose concentrations applied in the experimental conditions are obtained through serial dilution, transferring 1/10 total volume to each subsequent dilution (Figure 11).

Cells are seeded in each well in standard DMEM medium. Each condition will be performed in duplicate. After 24 hours, medium is replaced with UDP-galactose specific to the experimental condition, with negative control and galactose medium wells receiving standard DMEM medium and 2 mM galactose medium respectively. Medium is replaced after 48h hours. After 48 hours medium is removed and cells are washed with PBS. Cells are then harvested by scraping in ice-cold PBS and transferred to 15 mL tubes. Supernatant is removed after centrifugation and the cell pellet stored at -80°C. Glycosylation activity is evaluated by quantification of glycosylated ICAM1 protein expression and glycomics profiling using MALDI-TOF mass spectrometry analysis for each condition.

For assessment of optimal UDP-galactose/UDP-glucose supplementation ratios cells will be cultured and analyzed as described above. Seeding medium is replaced 24 hours after seeding with medium containing UDP-galactose/UDP-glucose ratios of 1/1, 3/1, 5/1, and 7/1 UDP-galactose/UDP-glucose. The combined molarity of both nucleotide sugars for each condition will remain equal to that of the lowest effective concentration UDP-galactose determined in the primary analysis.

### Primary analysis

The primary analysis will focus on identification of the lowest effective concentration of UDP-galactose for restoration of glycosylation. Restoration of glycosylation is assessed through glycan profiling for each UDP-galactose concentration and the positive and negative control conditions by MALDi-TOFF mass spectrometry, and by quantification of glycosylated ICAM1 through Western blot. UDP-galactose induced restoration of glycan profiles should indicate a relative reduction of truncated glycans comparable to the positive control (2mM D-galactose). ICAM1 Western blotting indicates increased expression of glycosylation ICAM1 to the level of the positive control.

### Secondary analysis

Following the identification of the lowest effective dose of UDP-galactose, the optimal ratios for combined UDP-galactose/UDP-glucose supplementation are analyzed. Restoration of glycosylation is considered effective at levels comparable to the positive control.

### Example 4: Using UDPgalactose: UDP-glucose in the treatment of PGM1-CDG

PGM1-CDG patients have a peak concentration of 0.75-2 mM galactose in blood on a dose of 1-1.5g/kg/day oral D-galactose therapy. This concentration is sufficient to improve their glycosylation defects when the therapy is administered for a period of at least 6 weeks. *In vitro* experiments in patients' fibroblast using 2 mM galactose added to the culture medium for 5 days lead to significant increase in cellular UDP-hexose pools and improved cellular glycosylation. While the enzyme GALE (galactose epimerase) interconverts UDP-galactose with UDP-glucose, these intracellular nucleotide sugar pools are not equal. *In vitro* D-Galactose administration increases both sugar nucleotide pools, but has a more pronounced effect on the UDP-galactose pool.

This Example investigates whether UDP-galactose can be used as a building block for Golgi related glycosylation, and whether UDP-glucose has an important role in the initiation of the transfer process of the lipid linked oligosaccharides to the protein in the ER.

### Methods

### Nucleotide sugar and hexose phosphate pool levels

PGM1 deficient patient (P) and control (C) fibroblasts were grown for 3 days in DMEM (5 mM glucose, 2 mM glutamine) in the presence or absence of varied concentrations of UDP-Gal, UDP-Glc or their combination. 100 µL of medium was collected on the first and the last day of the experiments and 900 µL of extraction buffer was added. Metabolites were extracted in 250 µL of extraction buffer. All samples were processed and sugars in cell lysates were analyzed by mass spectrometry (LC-MS) as described elsewhere (see, e.g., Radenkovic et al., Am. J. Human Gen., 104:835-46 (2019)).

### UDPgalactose: UDP-glucose cell proliferation assay

Cells were seeded in 48 well plates at 9.0^{∗}10³ cells per well. Cell proliferation assays were performed through continued phase-contrast microscopy using an IncuCyte live-cell imaging system (Essen BioScience, Ann Arbor, MI, USA). Cells were monitored for thirteen days in UDP-galactose supplementation conditions, and eight days in co-cultured UDP-galactose and UDP-glucose supplementation conditions. Cell proliferation images were obtained every 24 hours. Culture medium was replaced every 48 hours. Data were extracted from IncuCyte and processed using PRISM software (LaJolla, CA, USA). Statistical analysis was performed using a two-way ANOVA test. All conditions were tested in quadruplicate.

### Glycosylation Assay

Western blots were evaluated for cell surface glycosylation of the fibroblasts by ICAM1 analysis (Wong et al., Gen. in Med.: Off. J. Am. Coll. Med. Gen., 19:1226-35 (2017); and Radenkovic et al., Am. J. Human Gen., 104:835-46 (2019)).

### Results

The effect of using a low concentration of UDP-hexoses, instead of a high concentration of D-galactose in PGM1 deficiency, was evaluated, and whether these compounds (UDP galactose and UDP glucose), used in a specific ratio, would result in the same biochemical effects as using high concentration galactose *in vitro* was assessed.

### Nucleotide sugar and hexose phosphate pool levels

Patient (P3) and Control (C4) were cultured in standard glucose containing medium. UDP sugar pools were measured by mass spectrometry in cell lysates after 5 days of culture. Adding 5 µM UDP-galactose to the cultured cells increased UDP hexose pools with 20% in control cells and 30% in patient cells (Figure 12A). Adding 100 µM UDP-galactose to the culture increased UDP hexose pools with 30% in control cells and 40% in patient cells (Figure 12B). Adding 100 µM UDP-glucose to the culture increased UDP hexose pools with 30% in control cells and 40% in patient cells (Figure 12C).

Patient (P5) and Control (C4) fibroblasts were grown for 3 days in DMEM 5.5 mM glucose (glc), 2 mM glutamine in the presence or absence of 5 µM UDP-Galactose (UDP-Gal). The metabolites were extracted and analyzed by LC-MS. The observed relative abundance of UDP-Hexose compared to the untreated control in the presence of 5 µM UDP-Galactose was C4: 1.00>1.22, P5 0.86>1.08; and the relative abundance of UDP-HexNAc was C4: 1.00> 1.05, P5: 0.69> 0.77 (Figure 13).

Patient (P3) and Control (C4) fibroblasts were grown for 3 days in DMEM 5.5 mM glucose (glc), 2 mM glutamine in the presence or absence of 10 µM UDP-Galactose (UDP-Gal). The metabolites were extracted and analyzed by LC-MS. The observed relative abundance of UDP-Hexose compared to the untreated control in the presence of 10 µM UDP-Galactose was C4: 1.00>1.32, P3 0.46>0.62; and the observed relative abundance of UDP-HexNAc C4: 1.00> 1.33, P3: 0.7> 0.89 (Figure 14).

Patient (P3) and Control (C4) fibroblasts were grown for 3 days in DMEM 5.5 mM glucose (glc), 2 mM glutamine in the presence or absence of 10 µM UDP-Galactose (UDP-Gal) and 5 µM UDP-Glucose (UDP-Glc). The metabolites were extracted and analyzed by LC-MS. The observed relative abundance of UDP-Hexose compared to the untreated control in the presence of 10 µM UDP-Galactose (UDP-Gal) and 5 µM UDP-Glucose (UDP-Glc) was C4: 1.00>1.21, P3: 0.46>0.58; and the relative abundance of UDP-HexNAc was C4: 1.00> 1.2, P3: 0.7> 0.87 (Figure 15).

Patient (P3) and Control (C4) fibroblasts were grown for 3 days in DMEM 5.5 mM glucose (glc), 2 mM glutamine in the presence or absence of 10 µM UDP-Galactose (UDP-Gal) and 10 µM UDP-Glucose (UDP-Glc). The metabolites were extracted and analyzed by LC-MS. The relative abundance of UDP-Hexose compared to the untreated control in the presence of 10 µM UDP-Galactose (UDP-Gal) and 10 µM UDP-Glucose (UDP-Glc) was C4: 1.00>1.26, P3: 0.46>0.44; and the relative abundance of UDP-HexNAc was C4: 1.00> 1.22, P3: 0.7> 0.84 (Figure 16).

Patient (P6) and Control (C4) fibroblasts were grown for 3 days in DMEM 5.5 mM glucose (glc), 2 mM glutamine in the presence or absence of 100 µM UDP-Galactose (UDP-Gal). The metabolites were extracted and analyzed by LC-MS. The observed relative abundance of UDP-Hexose compared to the untreated control in the presence of 100µM UDP-Galactose were C4: 1.00>1.64, P6: 0.61>1.34; and the relative abundance of UDP-HexNAc was C4: 1.00> 1.48, P6: 0.9> 1.41 (Figure 17).

Patient (P6) and Control (C4) fibroblasts were grown for 3 days in DMEM 5.5 mM glucose (glc), 2 mM glutamine in the presence or absence of 100 µM UDP-Glucose (UDP-Glc). The metabolites were extracted and analyzed by LC-MS. The relative abundance of UDP-Hexose compared to the untreated control in the presence of 100 µM UDP-Glucose (UDP-Glc) was C4: 1.00>1.26, P3: 0.61>1.01; and the relative abundance of UDP-HexNAc was C4: 1.00> 1.14, P6: 0.9> 1.42 (Figure 18).

### Concentration of UDP-hexosesfor optimal cell growth of PGM1 deficient patient cells

Putatively detrimental effects of UDP-galactose supplementation on cell homeostasis were assessed through analysis of cell proliferation under various galactose compound concentrations. Screening for the highest tolerated UDP-galactose concentrations was performed in healthy control and PGM1-CDG patient fibroblast samples supplemented with 1 µM, 10 µM, 100 µM and 1000 µM UDP-galactose, and a positive control condition supplemented with 2 mM D-galactose previously shown to be safe and functionally relevant, as indicated by restored glycosylation in PGM1-patient derived fibroblast samples (see, e.g., Radenkovic et al., Am. J. Human Gen., 104:835-46 (2019); and Wong et al., Gen. in Med.: Off. J. Am. Coll. Med. Gen., 19:1226-35 (2017)). UDP-galactose compound screening indicated decreased cell proliferation at 1000 µM compared to control condition (2 mM galactose) (two-way ANOVA interaction p< 0.0001) but not for 1-100 µM UDP-galactose supplementation conditions in both patient and control cells (Figure 19).

Following establishment of safe UDP-galactose concentrations ranges of 1-100 µM, combined UDP-galactose and UDP-glucose supplementation conditions were assessed for potential added effects on cell proliferation. UDP-galactose: UDP-glucose concentrations of 10:7.5, 10:5, 10:2.5, and 10: 1 were tested. UDP-galactose concentrations were kept at 10 µM, co-supplemented with varying UDP-glucose concentrations between 1-7.5 µM. No difference between cell proliferation rates were observed between the tested conditions (Figure 20).

### Concentration of UDP-hexoses for optimal glycosylation in PGM1 deficient patient cells

Cells cultured in 4 mM glucose containing media were treated with adding 10 µM UDP-galactose, and in addition, adding different concentrations of UDP-glucose. Western blots were evaluated for cell surface glycosylation of the fibroblasts by ICAM1 analysis. There were no significant differences between the different treatment conditions (Figure 21).

### Conclusions

These results demonstrate that UDP sugar administration can increase cellular UDP sugar pools (5 µM UDP galactose can increase nucleotide sugar pools with 30%=comparable to the effect of 2 mM D-galactose in vitro), and that UDP hexoses between 5-100 µM are safe and efficient.

### Example 5: Treating PGM1-CDG

A human identified as having PGM1-CDG is administered UDP-glucose and UDP-galactose. For example, a human having PGM1-CDG can be treated by administering UDP-glucose and UDP-galactose at a ratio of about 7: 1. The administered inhibitor(s) can reduce the severity of one or more symptoms of PGM1-CDG.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

### EMBODIMENTS

Although the present invention is defined in the claims, it should be understood that the present invention can also (alternatively) be defined in accordance with the following embodiments:
1. A method for treating a mammal having a congenital disorder of glycosylation (CDG), wherein said method comprises administering a composition comprising UDP-galactose and UDP-glucose to said mammal.
2. The method of embodiment 1, wherein said mammal is a human.
3. The method of any one of embodiments 1-2, wherein said CDG is phosphoglucomutase 1 CDG (PGM1-CDG).
4. The method of any one of embodiments 1-3, wherein a ratio of UDP-galactose to UDP-glucose is from about 1:1 to about 10:1.
5. The method of embodiment 4, wherein a ratio of UDP-galactose to UDP-glucose is 3:1.
6. The method of any one of embodiments 1-5, wherein administering said composition results in said mammal having a blood concentration of UDP-galactose of from about 5 µM to about 50 µM.
7. The method of any one of embodiments 1-6, wherein administering said composition results in said mammal having a blood concentration of UDP-glucose of from about 2 µM to about 20 µM.
8. The method of any one of embodiments 1-7, wherein said treatment is effective to reduce one or more symptoms of said CDG.
9. The method of any one of embodiments 1-8, wherein said treatment is effective to restore glycosylation in said mammal.
10. A method for restoring glycosylation in a mammal having a congenital disorder of glycosylation (CDG), wherein said method comprises administering a composition comprising UDP-galactose and UDP-glucose to said mammal.
11. The method of embodiment 10, wherein said mammal is a human.
12. The method of any one of embodiments 10-11, wherein said CDG is phosphoglucomutase 1 CDG (PGM1-CDG).
13. The method of any one of embodiments 10-12, wherein a ratio of UDP-galactose to UDP-glucose is from about 1:1 to about 10:1.
14. The method of embodiment 13, wherein a ratio of UDP-galactose to UDP-glucose is 3:1.
15. The method of any one of embodiments 10-14, wherein administering said composition results in said mammal having a blood concentration of UDP-galactose of from about 5 µM to about 50 µM.
16. The method of any one of embodiments 10-15, wherein administering said composition results in said mammal having a blood concentration of UDP-glucose of from about 2 µM to about 20 µM.
17. The method of any one of embodiments 10-16, wherein said restored glycosylation is assessed using a ratio of a reduced transferrin glycosylation form over a normal transferrin glycosylation form, and wherein a decreased ratio indicates restored glycosylation.
18. The method of any one of embodiments 10-16, wherein said restored glycosylation is assessed using a level of one or more liver enzymes within said mammal, wherein a decreased level of one or more liver enzymes indicates restored glycosylation, and wherein said one or more liver enzymes are selected from the group consisting of aspartate transaminase (AST) and alanine transaminase (ALT).
19. The method of any one of embodiments 10-16, wherein said restored glycosylation is assessed using a level of one or more coagulation factors within said mammal, wherein a decreased level of one or more coagulation factors indicates restored glycosylation, and wherein said one or more coagulation factors comprise activated prothrombin time (aPTT).
20. The method of any one of embodiments 10-16, wherein said restored glycosylation is assessed using a level of one or more coagulation factors within said mammal, wherein an increased level of one or more coagulation factors indicates restored glycosylation, and wherein said one or more coagulation factors are selected from the group consisting of anti-thrombin III (ATIII), Factor XI, and Factor XIII.

## Claims

1. A composition comprising UDP-galactose and UDP-glucose for use in a method for treating a mammal having a congenital disorder of glycosylation (CDG), wherein said method comprises administering said composition to said mammal.

2. The composition for use of claim 1, wherein said mammal is a human.

3. The composition for use of any one of claims 1-2, wherein said CDG is phosphoglucomutase 1 CDG (PGM1-CDG).

4. The composition for use of any one of claims 1-3, wherein a ratio of UDP-galactose to UDP-glucose is from about 1:1 to about 10:1, optionally wherein a ratio of UDP-galactose to UDP-glucose is 3:1.

5. The composition for use of any one of claims 1-4, wherein administering said composition results in said mammal having a blood concentration of UDP-galactose of from about 5 µM to about 50 µM, and/or wherein administering said composition results in said mammal having a blood concentration of UDP-glucose of from about 2 µM to about 20 µM.

6. The composition for use of any one of claims 1-5, wherein said treatment is effective to reduce one or more symptoms of said CDG, and/or wherein said treatment is effective to restore glycosylation in said mammal.

7. A composition comprising UDP-galactose and UDP-glucose for use in a method for restoring glycosylation in a mammal having a congenital disorder of glycosylation (CDG), wherein said method comprises administering said composition to said mammal.

8. The composition for use of claim 7, wherein said mammal is a human.

9. The composition for use of any one of claims 7-8, wherein said CDG is phosphoglucomutase 1 CDG (PGM1-CDG).

10. The composition for use of any one of claims 7-9, wherein a ratio of UDP-galactose to UDP-glucose is from about 1:1 to about 10:1, optionally wherein a ratio of UDP-galactose to UDP-glucose is 3:1.

11. The composition for use of any one of claims 7-10, wherein administering said composition results in said mammal having a blood concentration of UDP-galactose of from about 5 µM to about 50 µM, and/or wherein administering said composition results in said mammal having a blood concentration of UDP-glucose of from about 2 µM to about 20 µM.

12. The composition for use of any one of claims 7-11, wherein said restored glycosylation is assessed using a ratio of a reduced transferrin glycosylation form over a normal transferrin glycosylation form, and wherein a decreased ratio indicates restored glycosylation.

13. The composition for use of any one of claims 7-11, wherein said restored glycosylation is assessed using a level of one or more liver enzymes within said mammal, wherein a decreased level of one or more liver enzymes indicates restored glycosylation, and wherein said one or more liver enzymes are selected from the group consisting of aspartate transaminase (AST) and alanine transaminase (ALT).

14. The composition for use of any one of claims 7-11, wherein said restored glycosylation is assessed using a level of one or more coagulation factors within said mammal, wherein a decreased level of one or more coagulation factors indicates restored glycosylation, and wherein said one or more coagulation factors comprise activated prothrombin time (aPTT).

15. The composition for use of any one of claims 7-11, wherein said restored glycosylation is assessed using a level of one or more coagulation factors within said mammal, wherein an increased level of one or more coagulation factors indicates restored glycosylation, and wherein said one or more coagulation factors are selected from the group consisting of anti-thrombin III (ATIII), Factor XI, and Factor XIII.
